# EUROPEAN PATENT APPLICATION

(11) **EP 2 455 456 A1**
(43) Date of publication of application: **23.05.2012**
(21) Application number: 10306280.8
(22) Date of filing: 22.11.2010
(51) Int. Cl.: C12N 9/14, C12N 15/113, A61K 31/7105, C12N 15/62, A61P 31/18, C12Q 1/34, G01N 33/68

(54) **Use of kinesin inhibitors in HIV infection treatment and a method for screening them**

(71) Applicant: Institut Curie, 75248 Paris Cedex 05 (FR)
(72) Inventor: The designation of the inventor has not yet been filed
(74) Representative: Gallois, Valérie

(57) **Abstract**

The present invention concerns new methods for treating HIV infection by using kinesin inhibitors and methods for identifying new molecules of interest for treating AIDS.

## Description

### Field of the invention

The present invention relates to the field of medicine and provides new therapeutic agents for treating HIV infection. The present invention relates to a kinesin inhibitor which is effective in the treatment of HIV infection and to methods of screening of molecules useful in the treatment of HIV infection, in particular using a tagged kinesin protein.

### Background of the invention

Human immunodeficiency 1 (HIV-1), identified in 1983 as the cause of acquired immune deficiency syndrome (AIDS), remains a global health threat responsible for a world-wide pandemic. CD4+ T lymphocytes play a central role in the pathogenesis of human immunodeficiency virus (HIV) infection. In viremic patients, these cells are the major cellular target for the virus: HIV multiplies intensively in this sub population during the acute phase of the disease and continues to propagate in these same cells during the clinical latency phase. The problem of drug resistance in the treatment of HIV infection has become an increasingly significant barrier in the effectiveness of AIDS chemotherapy. Despite the initial success of monotherapy for HIV infection with agents such as AZT (Zidovudine), the benefits are short lived. A key factor in HIV chemotherapy failure is the rapid selection by HIV for drug-resistant strains. It is observed that resistance develops rapidly to reverse transcription inhibitors such as AZT and other drugs that reduce viral production. The resistance is due to the ability of the virus to mutate and circumvent the normal transcription pathway which the chemotherapy interrupts (Volberding et al; 1990). To limit viral escape, treatments involving more than one anti-retroviral drug have then been developed. In 1996, a major advance has been made in curing AIDS with the introduction of the highly active antiretroviral therapy (HAART), which usually includes a combination of reverse transcriptase and protease inhibitors. (Carpenter, C. et al. Antiretroviral therapy for HIV infection in 1997. JAMA 277, 1962, 1997). Two types of RT inhibitors exist, namely nucleoside reverse transcriptase inhibitors (NRTIs) and non-nucleoside reverse transcriptase inhibitors (NNRTIs). Such HAART treatment has proved to be effective with a pronounced drop in viral load, increase in lymphocyte populations, reduction in opportunistic infections and a doubling of time of progression to serious illness and/or death.

However, the viral infection is contained with such treatment but not eliminated. Indeed, multiple drug resistance and long-term persistence of drug-resistant viral reservoirs have been reported, making the emergence of secondary epidemics of multi-drug resistant HIV-1 a real long-term threat.

These observations suggest that even long term suppression of HIV-1 replication by HAART cannot totally eliminate HIV-1. The virus persists in cellular reservoirs because of viral latency, cryptic ongoing replication or poor drug penetration, and the residual virus may, after months or years of persistence and low-level replication, develop resistance to the drugs and reemerge. The continuing emergence of drug resistance further threaten the future therapy, thereby necessitates another treatment strategy.

The production of new infectious retroviral particles is an ordered process that includes many steps. The various components of the virions have to be transported to the assembly site to form new viral particles.

In particular, three major viral components, Gag, the envelope and genomic RNAs have to traffic inside the cell to reach their assembly site. It has long been established that Gag is the key component driving the assembly process. Gag is a polyprotein synthesized in the cytosol as a Pr55Gag precursor, which will be cleaved once in the viral particle to give rise to the following four main polypeptides: Matrix (p17), Capsid (p24), Nucleocapsid (p7) and the p6 domain. Pr55Gag probably coordinates the recruitment of different host proteins necessary for at least its transport to the assembly site, and the formation of the viral particles.

It is known that HIV-1 encodes only 15 proteins and thus must exploit multiple host cell proteins for successful infection. Targeting host factors required for the HIV cycle represents an alternative pharmacological strategy for treating HIV-infected individuals as drugs targeting such host factors are much less likely to escape mutation that interfere with the effectiveness of conventional antiretroviral drugs.

In attempts to identify those host proteins needed for HIV infection, several genome wide screens using various techniques have been carried out including 9 genome-wide 3 independent siRNA screens, (PLos pathogens 5:e100000437 (2009), a genome-wide RNAi screen coupled to interrogation of human interactome network databases (Köning et al. Cell, 135:49 (2008) and a genome-wide short hairpin RNA screening (Yeung et al., JBC 284:19463 (2009). Overall, although a large number of potential host proteins (up to 2410) linked to HIV replication were identified, very poor overlap in the results were obtained .. In addition, it is worth noting that most of these genome-wide studies were performed in non-myeloid cells and most of the time in cell lines such as COS, HeLa and 293T cells with very different characteristics from those of primary infected CD4+ T lymphocytes or macrophages. Moreover, we are currently lacking functional experiments addressing the different roles of these proteins depending on cell type in contributing to the production of viral particles.

In T cells, the actin and tubulin cytoskeleton has been proposed to play an active role for HIV assembly (jolly, 2007 , 4525) (Nishi, 2009, 4456). In addition, in a genome wide screen using HEK293T cells, a protein transporting cargo proteins along microtubules, kinesin KIF3A, was found to be potentially involved in the HIV viral cycle probably through an interaction with the Tat viral protein (Konig, 2008, 4441). However, such result was never confirmed in any of the other genome-wide studies or by any other functional study.

As a matter of fact, little is indeed known about host cell proteins interacting with HIV in resting memory CD4+ T cells which are the best characterized reservoir in the natural host and even less in macrophages which can be infected latently in the lymph nodes or in haematopoitic stem cells of the bone marrow and therefore contribute to the viral persistence by the formation of new virions (Alexaki A, Liu Y, Wigdahl B: Cellular reservoirs of HIV-1 and their role in viral persistence. Curr HIV Res 2008, 6:388-400). Macrophages are relatively long-lived cells that are less prone to cell death as a result of HIV-1 infection than other cells and are therefore likely to be a major site of viral latency. With the half-lives of macrophages reported to be of the order of months to years, depending on the specific type of macrophage, and with reports of higher concentrations of antiviral drugs being required to inhibit HIV-1 replication in macrophages than in T cells, infected macrophages pose a serious problem for viral eradication with current antiretroviral therapy. In addition to the challenge of latently infected resting memory T cells, the problem of long-lived infected macrophages in the successful treatment of HIV-1 infection is highlighted by recently determined viral decay kinetics with current treatment regimens. The poor penetration of some antiretroviral drugs into the cerebrospinal fluid may result in the development of a sanctuary site, where macrophages, in addition to microglial cells, can result in persistent viremia. If HIV-1 infection is to be cured, new strategies that will eradicate these reservoirs will be required. Very little data relating to host proteins involved in the HIV viral cycle in macrophage are available. Indeed, no genome-wide screen in such cells has been performed so far on such cell type. In addition, the potential role of the cellular cytoskeleton and cytoskeletal-related proteins has not been investigated.

Accordingly, there is a significant need for alternative HIV treatments targeting host factors, especially host factors in viral reservoirs, in order to avoid immune viral escape.

### Summary of the invention

The present invention provides new therapeutic agents for treating HIV infection. The inventors have shown that the kinesin KIF3A plays a key role in the transport of Gag-containing viral compartments toward the cell periphery and that its silencing greatly reduces HIV virion release.

Therefore in a first aspect, the present invention concerns kinesin inhibitors, preferably N-type kinesin inhibitors, more preferably kinesin 1 inhibitors or kinesin 2 inhibitors, even more preferably KIF3A inhibitor, for use in the treatment of HIV infection.

In a second aspect, the present invention concerns a tagged kinesin comprising a kinesin motor domain, a stalk domain, a cargo domain and a tag allowing said tagged kinesin to be detected, and wherein said tag is located between said stalk domain and said cargo domain. In a third aspect, the present invention concerns a method for screening for compounds useful for the treatment of HIV infection, said method comprising the steps of:
a) providing or obtaining a candidate compound; and
b) determining whether said candidate compound inhibits the activity and/or expression of a kinesin, preferably a N-type kinesin, more preferably a kinesin-1 or a kinesin-2, even more preferably kinesin KIF3A, wherein the ability of said candidate compound to inhibit the expression or activity of said kinesin indicates that said candidate compound is indicative of its usefulness for the treatment of HIV infection.

### Brief description of the drawings

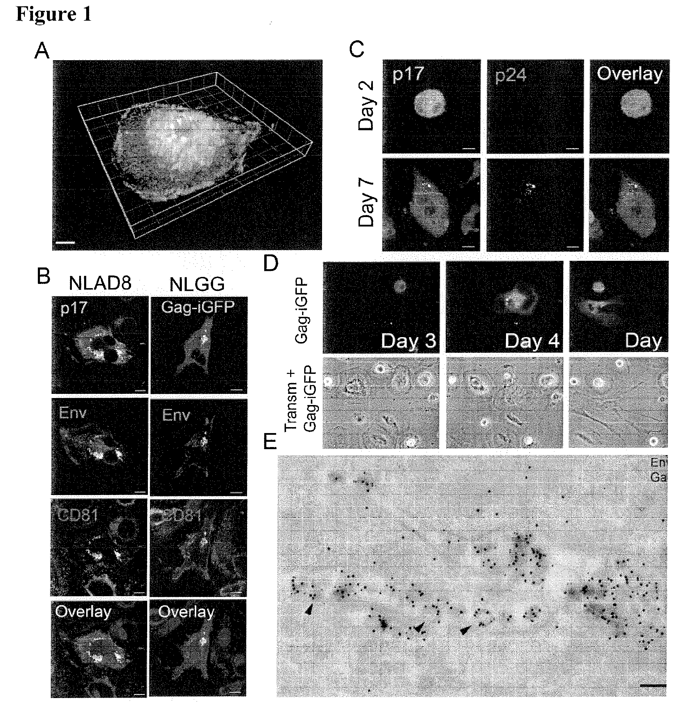

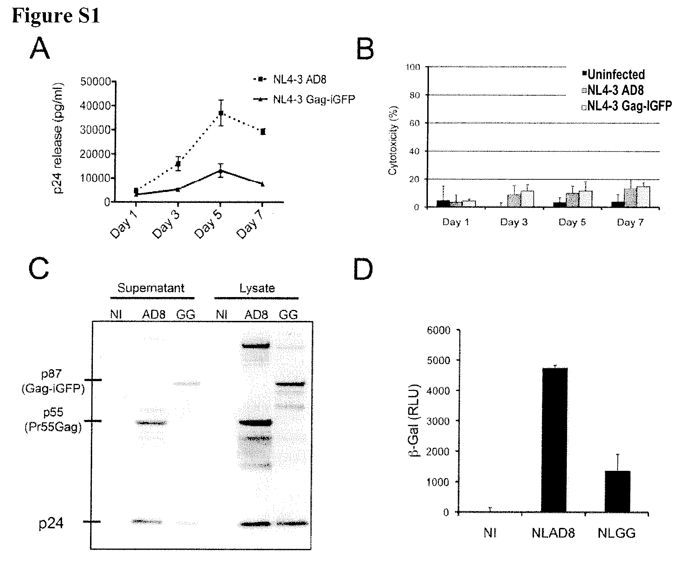

**Figure 1****.** Distribution and dynamics of Gag expression in macrophages following HIV infection
   (A) 3D reconstruction of macrophages infected with HIV-1 NLAD8 for 7 days and stained for Gag. Images were acquired by confocal microscopy and processed with the Imaris software.
   (B) Comparison of macrophages infected by NLAD8 or HIV Gag-iGFP for 7 days and stained for the indicated markers.
   (C) Macrophages were infected for the indicated number of days with NLAD8 and labeled with anti-p17 antibodies specific for both precursor and mature forms of Gag, and an anti-p24 mAb specific for the cleaved (mature) form of Gag. Bars, 10 µm.
   (D) Macrophages infected with HIV Gag-iGFP ΔEnv virus were imaged for 5 days from day 2 to day 5 p.i. Images were acquired every 10 min with an epifluorescent Biostation microscope. Here are presented 3 snapshots from the movie at the indicated days p.i.
   (E) Ultrathin cryosections of macrophages infected with HIV-1 NLAD8 for 15 days were prepared and labeled with anti-Pr55Gag antibodies and Protein A coupled to gold particles of 10 nm diameter (PAG10) and with anti-Env antibodies and PAG15. Bar, 200 nm.

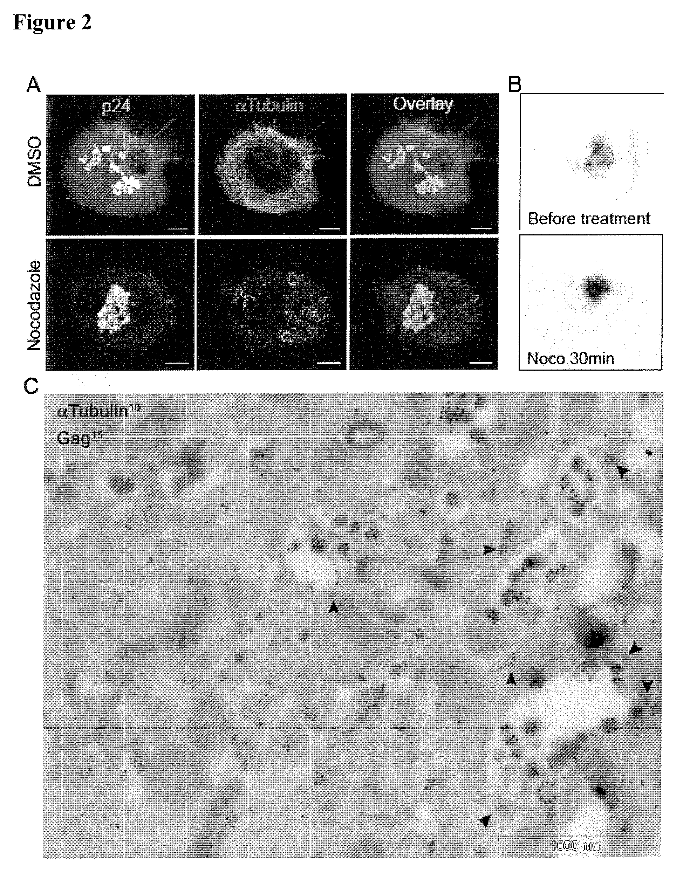

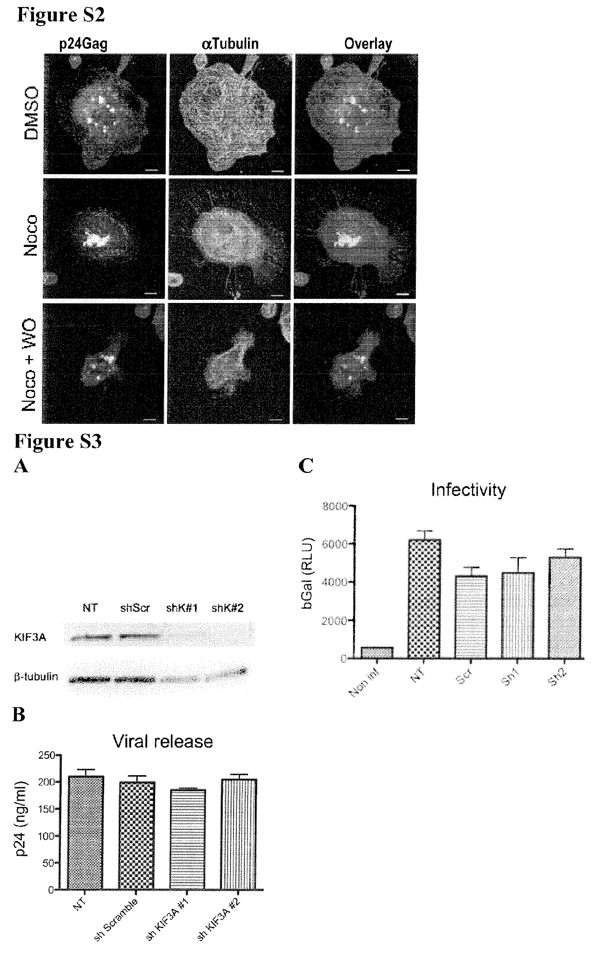

**Figure 2****.** Microtubule-dependant distribution of virus-containing compartments
   (A) Confocal micrographs of HIV-1-infected macrophages exposed to DMSO- or Nocodazole (10µM) for 1 hr. Cells were fixed and stained with antibodies specific for the indicated proteins (α-tubulin and p24 Gag). Gag+ compartments as well as cytosolic Gag were deeply affected and redistributed in a perinuclear area. Bar, 5 µm.
   (B) Time-lapse imaging of HIV-1-infected macrophages upon nocodazole exposure. Macrophages were infected with HIV Gag-iGFP virus for 7 days and imaged on an epifluorescent Biostation microscope for 2 hr. Two micrographs of the GFP fluorescence extracted from the movie are shown just before or 30 min after addition of Nocodazole.
   (C) Ultrathin cryosection of macrophages infected with HIV-1 NLAD8 for 15 days were double-immunogold labeled for Pr55Gag with PAG10 and for αtubulin with PAG15. Tubulin staining was present at the limiting membrane of virus-containing compartments (see arrows). Bars, 200 nm.

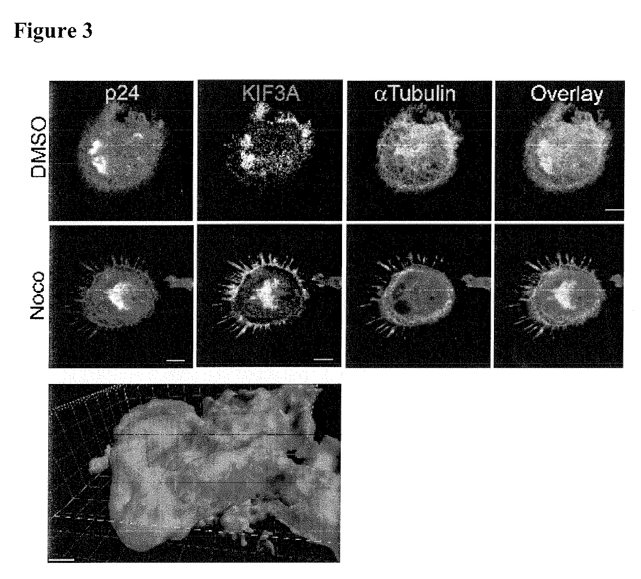

**Figure 3****.** The virus-containing compartments are associated with Tubulin and the kinesin KIF3A.
   Confocal micrographs of HIV-1-infected macrophages exposed to DMSO-
   (A) or Nocodazole (10µM)
   (B) for 1 hr. Cells were fixed and stained with antibodies specific for the indicated proteins (α-tubulin, KIF3A and p24 Gag). Bar, 10 µm.
   (C) 3D reconstruction of a Gag+ compartment (in green) and of the distribution of KIF3A (in red) from a stack of images acquired by confocal microscopy using the Imaris software.

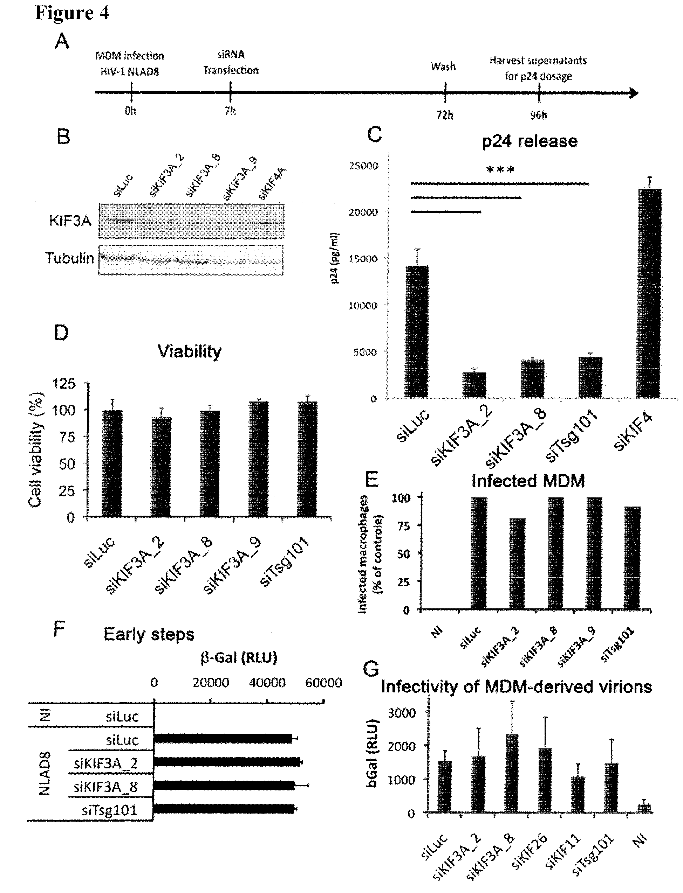

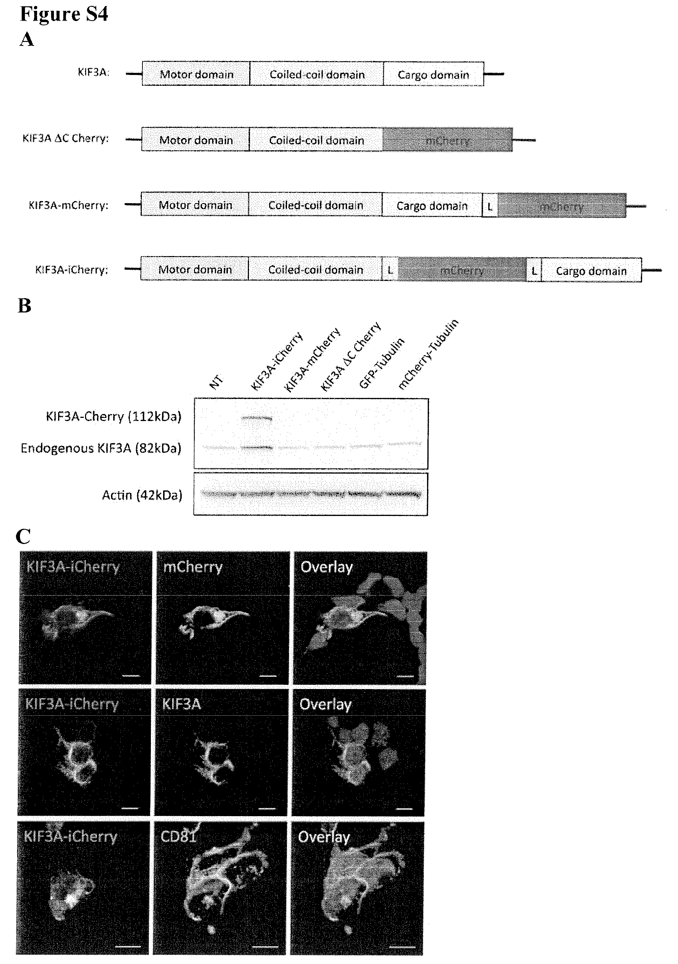

**Figure 4****:** KIF3A is required for viral production by macrophages but not in T lymphocytes
   (A) Schematic representation of the experimental design to perform knockdowns by siRNA transfection on HIV-1 infected macrophages.
   (B) Western blot analysis of KIF3A expression in macrophages transfected with the indicated siRNA. All siRNA specific for KIF3A reduced after 3 days its level of expression down to 20% in primary macrophages.
   (C) KIF3A depletion in macrophages reduced the amount of p24 release to the same extent as Tsg 101 depletion. *** indicates that the two histogram bars are significantly different (p value below 0.001).
   (D) Cell viability was measured using the CellTiter glo kit and normalized to the control (siLuc) cytotoxicity.
   (E) Percentages of infected macrophages were determined by flow cytometry.
   (F) Impact of the KIF3A knock down on the early steps of infection. The reported cell line TZM-bl was first transfected with the indicated siRNA, infected with HIV-1 for 24 hr and then β-gal activity was measured.
   (G) Infectivity of the virions produced by the macrophages submitted to the indicated siRNA was evaluated (see Material and Methods).

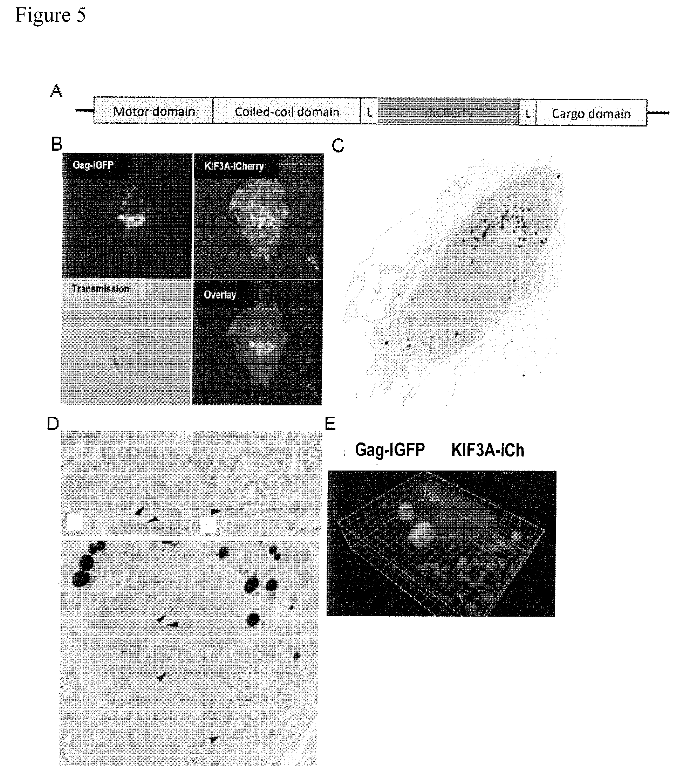

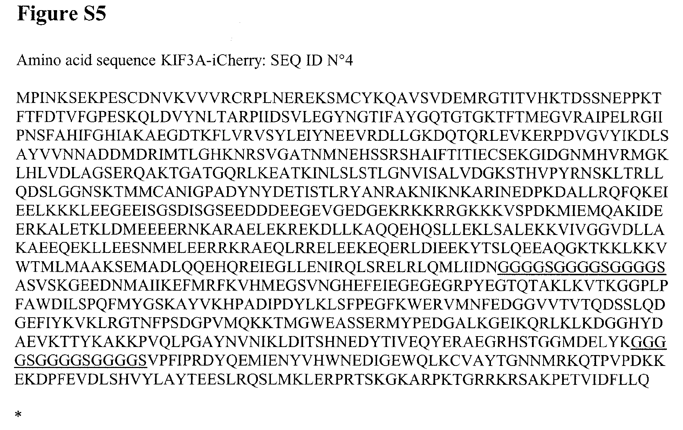

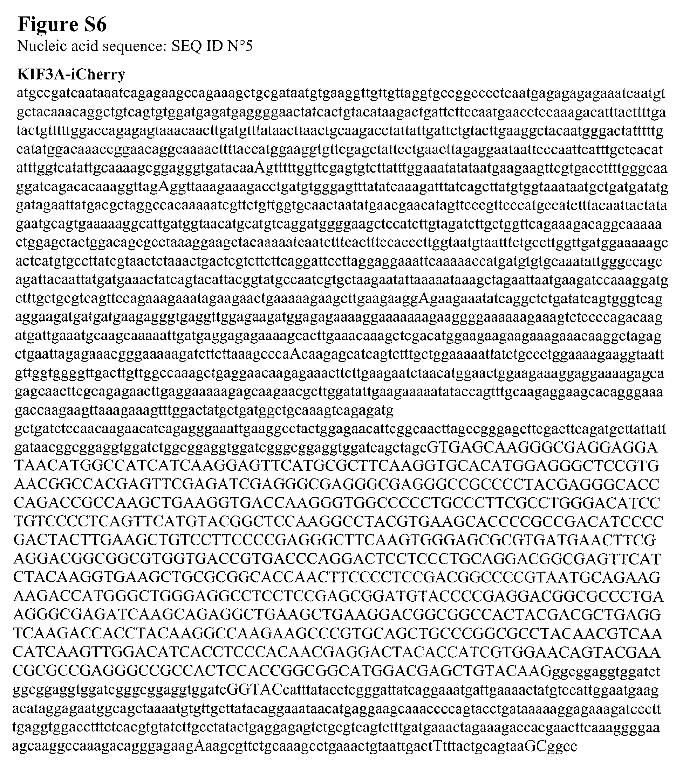

**Figure 5****:** Intracellular trafficking of the virus-containing compartments is driven by KIF3A
   (A) Schematic representation of the KIF3A-iCh construct.
   (B to D) Purified monocytes were plated with M-CSF on CELLocate coverslips that etched grids with coordinates. After 7 days, macrophages were co-infected with HIV-1 Gag-iGFP and KIF3A-iCherry lentiviral vector. At day 7 p.i., cells were fixed and imaged by confocal microscopy. Then, coverslips were embedded in epon and processed for electron microscopy.
   (E) Time 0 of a 3D reconstruction carried out from time-lapse imaging by spinning disc confocal microscopy of macrophages co-infected with HIV-1 Gag-iGFP ΔEnv and KIF3A-iCherry lentiviral vector and cultured for 5 days.

### SUPPLEMENTAL INFORMATION

### Figure S1. HIV Gag-iGFP characterization

Monocyte derived macrophages were infected with HIV1 NLAD8 (AD8) or with HIV Gag-iGFP (GG). (A) Amounts of p24 released in the culture supernatant were determined by ELISA at various times after infection. HIV Gag-iGFP-infected cells produced significant but lower amounts of p24 than NLAD8-infected cells. The kinetics of p24 production was similar in both conditions. (B) Cytotoxicity was measured in both types of culture at the indicated times post infection. (C) Western blot analysis of HIV proteins revealed with IVIG (AIDS Research and Reference Reagent Program) and present in the supernatant and cell lysates of macrophage cultures not infected (NI), NLAD8-infected, HIV Gag-iGFP-infected (NLGG). The Gag-iGFP fusion protein was well expressed in macrophages and in virions secreted and was well processed into p24. (D) Infectivity assay using the TZM-bl reporter cell line of the virions produced by infected macrophages. HIV Gag-iGFP produced by macrophages were infectious although less than NLAD8. We concluded that HIV Gag-iGFP behaved like normal HIV virus although its production and infectivity were lower.

### Figure S2. The distribution of Gag+ compartments depends on the integrity of the microtubule network.

Confocal micrographs of HIV-1-infected macrophages exposed to DMSO- or Nocodazole (10µM) for 1 hr (Noco) or Nocodazole for 1 hr followed by washes and re-incubation for 15 min at 37°C (Noco + WO). Cells were fixed and stained with antibodies specific for the indicated proteins (α-tubulin and p24 Gag). Upon Nocodazole exposure, Gag+ compartments were redistributed in the perinuclear area. This effect was reversible as upon wash out of the Nocodazole, microtubule growth was accompanied by spreading of the Gag+ compartments.

### Figure S3. KIF3A knockdown does not affect virus production in T cells.

JLTRG cells were infected or not with lentivirus carrying shRNA scramble or against KIF3A. After puromycin selection, cells were infected with HIV for 8 hr and washed. Supernatant was collected 16 hr after wash. (A) Cell lysates were used for western blot with anti-KIF3A and anti-b-tubulin. Knockdown was quantified in relation to untreated cells and corresponded to approximately 57% and 93% (for shKIF3A #1 and #2, respectively). (B) p24 contents in the supernatant was measured by ELISA. Values represented here have been corrected by the number of infected cells in each sample, as calculated using CellTiter-Glo (for number of cells) and GFP expression (for percentage of infection). (C) Infectivity assay was performed by incubating supernatant collected from JLTRGs containing equal amounts of p24 (2ng) with TZM-bl cells.

Methods: JLTRG are Jurkat T-derived cells which have been stably transfected with an LTR-GFP construct and were obtained through the AIDS Research and Reference Reagent Program, Division of AIDS, NIAID, NIH: JLTRG (Cat #11587), from Dr. Olaf Kutsch, (Kutsch O, et al. Antimicrob Agents Chemother. 2004; 48(5):1652-63). JLTRG cells were infected with lentivirus carrying shRNA scramble (MISSION Non-Target shRNA Control Vector, Sigma-Aldrich, catalogue number SHC002) or against KIF3A (MISSION shRNA, Sigma-Aldrich. Sh1 TRC number TRCN0000116812, Sh2 TRC number TRCN0000116814) and selected with puromycin (3 µg/ml). Similar numbers of untreated or lentivirus-infected JLTRG were washed and infected with HIV NL-AD8 VSV-g for 8 hr in puromycin-free medium, washed in PBS and cultured for 16 hr, when supernantant was collected for p24 quantification and infectivity assay. Cells were also harvested and used for cell quantification by CellTiter-Glo (Promega), lysed for western blot assay or fixed for flow cytometric analysis.

### Figure S4. Characterization of the KIF3A-iCherry construct

(A) Schematic representation of the various KIF3A constructs carried out. (B) 293T cells were transfected with the indicated constructs and analyzed by immunoblot revealed by antibodies specific for KIF3A. (C) Confocal micrographs of 293T cells expressing KIF3A-iCh and stained for the indicated proteins.

### Figure S5. Amino acid sequence KIF3A-iCherry.

Spacers are underlined. Cherry protein in grey. KIF3A motor domain is between amino acids N°s 1-350; KIF3A stalk domain between amino acid N°s 351-594; KIF3A cargo domain between amino acids N°s 862-968 of SEQ ID N°4, spacers between amino acids 595-609 and 847-861 and iCherry protein between amino acid 610-846.

### Figure S6._Nucleic acid sequence: SEQ ID N°5 KIF3A-iCherry.

The coding sequence is indicated in capital letters.

### Detailed description of the invention

The inventors showed, in a surprising way, that kinesin depletion, in particular KIF3A depletion, led to a strong reduction of HIV virion production in primary macrophages infected with full-length HIV. The HIV production could indeed be decreased by means of an inhibitor of kinesin, in particular by means of a nucleic acid blocking the expression of KIF3A. They indeed observed, using 3 different siRNA that had been validated in Hela cells by studying KIF3A protein expression by Western blot, that the depletion of KIF3A in primary macrophages led to a strong reduction (75%) of the amounts of HIV secreted in the supernatant. Depletion of another kinesin, KIF5B, also resulted in reduction of secreted HIV virions. The inventors thus revealed that inhibiting the expression and/or activity of a host factor, KIF3A or KIF5B, could decrease or inhibit HIV production by infected macrophages.

### 1. Kinesine inhibitors as therapeutic agents in HIV infection.

In a first aspect, the present invention provides new therapeutic agents for treating HIV infection, namely kinesin inhibitors and in particular KIF3A inhibitors or KIF5B inhibitors.

The term "HIV infection" defines a condition caused by the human immunodeficiency virus (HIV). Infection with HIV-1 is associated with a progressive decrease of the CD4⁺ T cell count and an increase in viral load. The stage of infection can be determined by measuring the patient's CD4⁺ T cell count, and the level of viral load in the blood. HIV infection has four basic stages: incubation period, acute infection, latency stage and AIDS. The initial incubation period upon infection is asymptomatic and usually lasts between two and four weeks. The second stage, acute infection, lasts an average of 28 days and can include symptoms such as fever, lymphadenopathy, pharyngitis, rash, myalgia, malaise, and mouth and esophageal sores. The latency stage, which occurs third, shows few or no symptoms and can last anywhere from two weeks to twenty years and beyond. AIDS, the fourth and final stage of HIV infection is characterized by a very low level of CD4+ T cells and a severe immunodeficiency, and is accompanied by various opportunistic infections. In the context of the invention, the terms "treatment", "treat" or "treating" are used herein to characterize a therapeutic method or process that is aimed at (1) slowing down or stopping the progression, aggravation, or deterioration of the symptoms of the disease state or condition to which such term applies; (2) alleviating or bringing about ameliorations of the symptoms of the disease state or condition to which such term applies; and/or (3) reversing or curing the disease state or condition to which such term applies.

As used herein, the term "subject" or "patient" refers to an animal, preferably to a mammal, even more preferably to a human, including adult, child and human at the prenatal stage. However, the term "subject" can also refer to non-human animals, in particular mammals such as cats, horses, and non-human primates, among others, that are in need of treatment.

In the present description, the terms "nucleic acid," "nucleic sequence," "polynucleotide," "oligonucleotide" and "nucleotide sequence" are used interchangeably and refer to a sequence of deoxyribonucleotides and/or ribonucleotides.

As used herein the term "polypeptide" refers to any chain of amino acids linked by peptide bonds, regardless of length or post-translational modification. Polypeptides include natural proteins, synthetic or recombinant polypeptides and peptides (i.e. polypeptides of less than 50 amino acids) as well as hybrid, post-translationally modified polypeptides, and peptidomimetic.

As used herein, the term "amino acid" refers to the 20 standard alpha-amino acids as well as naturally occurring and synthetic derivatives. A polypeptide may contain L or D amino acids or a combination thereof.

As used herein the term "peptidomimetic" refers to peptide-like structures, which have non-amino acid structures substituted but which mimic the chemical structure of a peptide and retain the functional properties of the peptide. Peptidomimetics may be designed in order to increase peptide stability, bioavailability, solubility, etc.

All kinesins share a conserved core motor domain implying a common mechanism for generating force from ATP hydrolysis. Kinesins belong to a class of functional proteins generally called "molecular motors" and represent a superfamily of 15 classes including kinesin-related proteins of various functions.

Kinesins are a large superfamily of microtubule motor proteins that use the energy of ATP hydrolysis to produce force. They are defined by the presence of a catalytic core motor domain (historically known as the 'head' domain), which hydrolyzes ATP and binds to microtubules (MTs). Kinesins are classified into three subfamilies based on the position of their motor domain within the primary sequence of the protein. The Kin C (Kinesin 14) subfamily comprises kinesins with a C-terminally located core motor domain; Kin N kinesins (Kinesin-1 to 12) have an N-terminally located core motor domain; and Kin M (Kinesin 13) kinesins possess an internally located core motor domain. (Nobutaka Hirokawa et al, (2009) Kinesin superfamily motor proteins and intracellular transport) All kinesins share a high degree of sequence identity within the core motor domain. The crystal structures of the Kin N and Kin C core motor domains are clearly similar to each other. Functionally, the position of a kinesin's motor domain within the polypeptide chain usually predicts its directionality. Kin N kinesins walk toward the plus ends of MTs, whereas Kin C kinesins translocate toward the minus ends of MTs. Unexpectedly, the Kin I kinesins are not able to translocate along MTs in the conventional sense but, instead, depolymerize MT filaments from both ends. Kinesin is an oligomeric complex composed of two heavy chains and two light chains. The heavy chain is composed of three structural domains: a large globular N-terminal domain which is responsible for the motor activity of kinesin (it is known to hydrolyse ATP, to bind and move on microtubules), a central alpha-helical coiled coil domain, the stalk domain, that mediates the heavy chain dimerisation; and a small globular C-terminal domain, the cargo domain, which interacts with other proteins (such as the kinesin light chains), vesicles and membranous organelles.

As used herein in the context of the invention, the terms "kinesin", and KIF used interchangeably throughout the specification refer to all motor-type kinesins including both N-type and C-type kinesins but excluding kinesins unable to transport cargo proteins along microtubules. In particular, such terms refer to all naturally-occurring forms of motor kinesins including allelic variants, splice variants and isoforms.

In the context of the invention, we focus essentially on kinesins belonging to the kin N family, so-called "N-kinesin" or "N-type kinesins", more particularly on kinesins belonging to either the kinesin-1 and/or kinesin-2 subfamilies, even more particularly to KIF3A and KIF5B.

The KIF5 motor complex consists of two kinesin-1 family KIF5 motors, which are also known as kinesin heavy chains (KHCs). Occasionally, two kinesin light chains (KLCs) associate with the tail domains of the two KIF5 motors. Gene duplication has led to three subtypes of KIF5 in mammals, KIF5A, KI5B and KIF5C.

Kinesin-2 subfamily is one of the most ubiquitously expressed KIFs and has been implicated in the intracellular transport of membrane-bound organelles and protein complexes in various tissues such as neurons, melanosomes, and epithelial cells. Kinesin-2 molecules are expressed ubiquitously and transport cargoes along microtubules (MTs) from the nucleus to the cell periphery.

Kinesin-2 is a heterotrimeric complex composed of a KIF3A/3B heterodimer and KIF-associated protein 3 (KAP3 also known as KIFAP3). The KIF3A/3B heterodimer possesses a plus-end-directed microtubule sliding activity that uses energy derived from ATP hydrolysis. On the other hand, KAP3 links KIF3A/3B with various cargo proteins. KIF3A can form heterodimers either with KIF3B or KIF3C to walk along microtubules towards their plus end (Yamakasi, 1995 4547) (Yang, 1998 4548). KIF3A comprises of an N-terminal head (motor) domain that attaches to and migrates along MTs and C-terminal tail that presumably functions as the cargo-binding domain. KIF 17, an NH2-terminal motor domain-type motor, is also a member of the kinesin-2 family.

As used herein in the context of the invention, the terms "kinesin-2", "kinesins 2", "kinesin II" or "kinesin-2 family", used interchangeably throughout the specification, refer to all kinesins belonging to the kinesin 2 subfamily including KIF3A, KIF3B, KIF17 (also known as OSM3) and KAP3. Unigene Cluster for kinesin 2 include KIF3A, KIF3B,which representative mRNA and protein sequences are AB002357, and 015066 , respectively; KIF17, which representative mRNA and protein sequences are AY48442, and Q9P2E2 respectively; KAP3, which representative mRNA and protein sequences are M31158 and P31323 respectively.

As used herein, the terms "kinesin 3A", "KIF3A" and " Kinesin-like protein KIF3A" are used interchangeably throughout the specification and refer to all naturally-occurring forms of KIF3A including allelic variants, splice variants and isoforms. The Unigene Cluster for Kif3A is Hs AC004039, representative mRNA and protein sequences are AF041853, and Swissprot Q9Y496 (KIF3A_HUMAN), respectively.

As used herein in the context of the invention, the terms "kinesin-1", "kinesins 1", "kinesin I" or "kinesin-1 family", used interchangeably throughout the specification, refer to all kinesins belonging to the kinesin 1 subfamily (see Hirokawa et al., Mol. Cell biol. (2009), 10:682) including but not limited to KIF5A, KIF5B, and KIF5C.

As used herein, the term "kinesin inhibitor" refers to any molecule able to decrease or inhibit the expression and/or activity of a kinesin of interest according to the invention. Preferably, such a kinesin inhibitor is a direct inhibitor, meaning that it interacts directly with either a kinesin protein or a nucleic acid encoding a kinesin.

In one embodiment, the kinesin inhibitor is selective for a given kinesin of interest, preferably for KIF3A or for KIF5B, and/or a subset of kinesins, preferably N-type kinesins, more preferably kinesins 1 or kinesins 2. Are also encompassed Selectivity means that the kinesin inhibitor inhibits preferentially the expression and/or activity of the kinesin of interest (or subgroup of kinesins), or with a greater efficacy compared to other kinesins (or groups or kinesins), for instance by a factor of at least 10, 100 or 1000. If an inhibitor is selective for kinesin Kif3A, meaning it binds Kif3A with a greater efficacy compared to other kinesins, it is referred to throughout the specification as a Ki3fA selective inhibitor. Similarly inhibitors selective for either N-type kinesin or kinesin 2 are referred to as N-type kinesin selective inhibitors and kinesin 2 selective inhibitors respectively.

In a preferred embodiment of the invention, said kinesin inhibitor is a selective kinesin inhibitor, preferably a N-kinesin selective inhibitor, more preferably a kinesin 2 selective inhibitor or a kinesin 1 selective inhibitor. In an even preferred embodiment, said inhibitor is a KIF3A selective inhibitor or a KIF5B selective inhibitor.

However, in another embodiment, the kinesin inhibitor of the invention is an inhibitor able to inhibit the expression and/or activities of several kinesins of interest. Such multiple-specificity kinesin inhibitor can be useful especially in cases where Gag could be transported on microtubules by more than one kinesin. For example, kinesin inhibitors showing selectivity for both KIF3A and KIF5B are contemplated.

A kinesin inhibitor of the invention may act by blocking and/or inhibiting the activity of the kinesin of interest, preferably the activity of N-type kinesins, more preferably the activity of a kinesin 1 or a kinesin 2, even more preferably the activity of KIF5B or KIF3A.

This may for example be achieved by interfering with the motor activity of the kinesin of interest. Alternatively, this may be achieved by interfering with the binding of the kinesin of interest with its cargo protein. Both options ultimately result in blocking or reducing transport of the cargo protein, hence in blocking or reducing the kinesin functional activity. The kinesin inhibitors according to the invention are capable of inhibiting or decreasing the functional activity of the kinesin *in vivo* and/or *in vitro.* The inhibitor may inhibit the functional activity of the kinesin by at least about 10%, preferably by at least about 30%, preferably by at least about 50%, preferably by at least about 70, 75 or 80%, still preferably by 85, 90, 95, or 100%.

Functional activity of the kinesin may be readily assessed by the one skilled in the art according to known methods. Indeed, since, MT activated kinesin ATPase is a major parameter in kinesin motor function, functional activity of a kinesin may be assessed by measuring the level of ATPase activity. For example, Kinesin activity can be tested using the Kinesin ELIPA™ (Enzyme Linked Inorganic Phosphate Assay) Biochem Kit. The assay is an adaptation of a method originally described by Webb, M. R. (Pathway of the microtubule-kinesin ATPase. Biophys. (1992)) for the measurement of glycerol kinase plus D-glyceraldehyde ATPase activity and for actin activated myosin ATPase. The assay is based upon an absorbance shift (330 - 360 nm) that occurs when 2-amino-6-mercapto-7-methylpurine ribonucleoside (MESG) is catalytically converted to 2-amino-6-mercapto-7-methyl purine in the presence of inorganic phosphate (Pi). The reaction is catalyzed by purine nucleoside phosphorylase (PNP). One molecule of inorganic phosphate will yield one molecule of 2-amino-6-mercapto-7-methyl purine in an essentially irreversible reaction. Thus, the absorbance at 360 nm is directly proportional to the amount of Pi generated in the kinesin ATPase reaction.

Functional activity of a kinesin of interest may also be assessed by measuring its ability to transport a cargo protein, along microtubules. For example, the method described in Example 5 of the present invention may be used wherein a cargo protein tagged with GFP is coexpressed with a tagged kinesin (i.e. KIF3A-iCh) and the movements and eventual colocalization of both tagged proteins are determined using time lapse confocal microscopy. Analysis of the respective movements of both proteins in various conditions allows to characterize actual kinesin-dependent transport of the cargo protein.

The kinesin inhibitor of the invention may also act by blocking and/or inhibiting the expression of the kinesin of interest, preferably the expression of N-type kinesins, more preferably the expression of a kinesin 2 or of a kinesin 1; even more preferably the expression of Kif3A or KIF5B.

The decrease or the inhibition of kinesin expression can be evaluated by any means known to those skilled in the art including but not limited to assessing the level of the kinesin protein of interest using Western Blot analysis, for example using an Anti KIF3A antibody (Abcam), and assessing the level of mRNA for the kinesin of interest using any available technique such as qPCR for example. By a "decrease" in expression is meant, for example, a decrease of at least 30%, preferably at least 50%, more preferably at least 70%, or 80%, even more preferably at least 90%, 95% or 100% of the gene expression product.

Accordingly, a kinesin inhibitor according to the invention may be identified as a molecule which reduces the level of activity and/or expression of the kinesin of interest or subgroup of kinesins of interest using any of the methods and assays above mentioned and comparing the activity and/or expression of the kinesin(s) of interest in presence of the molecule whose kinesin inhibiting activity is to be assessed with the expression and/or activity of the kinesin(s) of interest in the absence of any such molecule.

Of particular interest, a kinesin inhibitor according to the invention may be further assessed for its ability to prevent Gag transport along microtubules and/or HIV virions release by any technique known to those skilled in the art including those described in the Example section of the present specification.

The kinesin inhibitor of the invention can be of various structural nature and include, without being limiting thereto, small molecules, aptamers, antibodies, nucleic acids, lipids, and peptides, polypeptides or proteins, such as kinesin dominant negative peptides or proteins, able to decrease kinesin expression and/or activity. The kinesin inhibitor may for instance be a peptide or polypeptide, such as an antibody or aptamer directed against the kinesin of interest; a nucleic acid molecule which reduces or prevents kinesin expression, such as an aptamer, a kinesin anti-sense oligonucleotide, a kinesin interfering RNA (iRNA) such as siRNA, or a ribozyme; or a small molecule inhibitor of kinesin activity.

The kinesin inhibitor of the invention is preferably selected from the group consisting of a small molecule, an anti-kinesin antibody, and a nucleic acid molecule interfering specifically with kinesin expression.

In an even preferred embodiment, said kinesin inhibitor is a kinesin siRNA, a kinesin antisens nucleic acid or a ribozyme interfering specifically with kinesin expression.

### 1.1 Inhibitors of kinesin activity

It is known that Pr55Gag coordinates the recruitment of different host proteins necessary for at least its transport to the assembly site and the formation of the viral particles. The inventors have shown that KIF3A is involved in the transport of Gag+ compartments containing virions.

Therefore the invention relates to the use of kinesin activity inhibitor, in particular KIF3A and/or KIF5B inhibitors, in HIV treatment.

The kinesin inhibitor of the invention can act through direct binding to a kinesin molecule. In this case, the inhibitor can be an antagonist which binds to the cargo binding domain or to another domain of the molecule which modifies the activity of the kinesin by steric hindrance or modification. This inhibitor can be, for instance, a small molecule, an aptamer, a dominant negative peptide, or an antibody directed against the active domain of the kinesin.

In one embodiment, a kinesin inhibitor according to the invention may be a small molecule inhibiting the activity of the kinesin(s) of interest. As used herein, the term "small molecule inhibiting the kinesin activity" refers to small molecule that can be an organic or inorganic compound, usually less than 1000 daltons, with the ability to inhibit or reduce the activity of the kinesin. Structural design in chemistry might help to find such a small molecule.

Small kinesin-inhibiting molecules are also known in the art and include, without being limiting thereto, tetracaïne, Lidocaïne, acepromazine, chlorfenethazine, chlorpromazine, N-methyl chlorpromazine, cyamemazine, fluphenazine, mepazine, methotrimeprazine, methoxypromazine, norchlorpromazine, perazine, perphenazine, phenothiazine, prochlorperazine, promethazine, propiomazine, putaperazine, thiethylperazine, thiopropazate, thioridazine, trifluoperazine, triflupromazine as well as inhibiting molecules described in patent applications and patents WO 01/98278, WO 02/057244, WO 02/079169, WO 02/057244, WO 02/056880, WO 03/050122, WO 03/050064, WO 03/049679, WO 03/049678, WO 03/049527, WO 03/079973, US 6,890,933, WO 2008/070739, WO 2006/060737, WO 2006/119146 and US 6,777,200.

In another embodiment, the kinesin inhibitor is an aptamer. The aptamers are nucleic acids, preferably RNA, generally comprising between 5 and 120 nucleotides (Osborne et al., 1997, Curr Opin Chem Biol. 1, 5-9).

As used here, the term "aptamer" means a molecule of nucleic acid or a peptide able to bind to a kinesin of interest or to a subgroup of kinesins of interest. It refers to a class of molecule that represents an alternative to antibodies in term of molecular recognition. Aptamers are oligonucleotide or oligopeptide sequences with the capacity to recognize virtually any class of target molecules with high affinity and specificity.

Such ligands may be isolated through Systematic Evolution of Ligands by EXponential enrichment (SELEX) of a random sequence library, as described in Tuerk C. and Gold L., Science, 1990, 249(4968):505-10. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., Clin. Chem., 1999, 45(9):1628-50. Peptide aptamers consist of a conformationally constrained antibody variable region displayed by a platform protein, such as E. coli Thioredoxin A that are selected from combinatorial libraries by two hybrid methods (Colas et al., Nature, 1996,380, 548-50).

As used here, the term "kinesin dominant negative peptide" refers to a peptide comprising at least part of a kinesin interacting with a cargo protein and able to suppress or decrease its activity.

In a preferred embodiment, the dominant negative peptide is only made up of the part of kinesin interacting with the protein cargo, i.e the part corresponding to the cargo domain of kinesin. This peptide is thus to interact with kinesin but, in the absence of the segment corresponding to the motor domain of kinesin, is unable to move along microtubules and thus to fulfill its function. Example of a KIF3A dominant negative peptide would consists essentially of or consists of the amino acid acids between positions 862 to 968 of SEQ ID N°4.

The inhibitor can be a specific antibody of the kinesin(s) of interest, preferably a N-type kinesins, more preferably a kinesin 1 or a kinesin 2, even more preferably KIF3A or KIF5B.

As used in the present invention, the term "antibody" includes monoclonal antibodies, chimeric antibodies, humanized antibodies, recombinant antibodies and fragments thereof. Antibody fragment means, for example F(ab)2, Fab, Fab' or sFv fragments. This small molecule can be derived from any known organism (including, but not limited to animals, plants, bacteria, fungi and viruses) or from a library of synthetic molecules immunologic binding agent such as IgG, IgM, IgA, IgD and IgE, and humanized or chimeric antibody. In certain embodiments, IgG and/or IgM are preferred because they are the most common antibodies in the physiological situation and they are most easily manufactured. The term "antibody" is used to refer to any antibody-like molecule that has an antigen binding region, and includes antibody fragments such as Fab', Fab, F(ab')₂, single domain antibodies (DABs), Fv, scFv (single chain Fv), and the like. The techniques for preparing and using various antibody-based constructs and fragments are well known in the art. Means for preparing and characterizing antibodies are also well known in the art (See, e.g., Harlow and Lane, 1988).

A "humanized" antibody is an antibody in which the constant and variable framework region of one or more human immunoglobulins is fused with the binding region, e.g. the CDR, of an animal immunoglobulin. "Humanized" antibodies contemplated in the present invention are chimeric antibodies from mouse, rat, or other species, bearing human constant and/or variable region domains, bispecific antibodies, recombinant and engineered antibodies and fragments thereof. Such humanized antibodies are designed to maintain the binding specificity of the non-human antibody from which the binding regions are derived, but to avoid an immune reaction against the non-human antibody.

A "chimeric" antibody is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity such as Mouse anti KIF3A (Becton Dickinson) .

### 1.2 Inhibitors of kinesin expression

The inhibition can also be due to the reduction or suppression of the expression of the gene product coding for the kinesin of interest, for example by using specific siRNA, antisense or ribozyme, which ultimately induce a decrease of the protein expression.

In a preferred embodiment of the invention, the kinesin inhibitor is a nucleic acid comprising or consisting of a sequence capable of hybridizing specifically with a nucleic acid (for example a gene or a mRNA) coding for a kinesin, preferably a N kinesin, more preferably a kinesin 2 or a kinesin 1, even more preferably KIF3A or KIF5B, and to decrease or suppress the expression of said kinesin. Such nucleic acids are more amply detailed below.

In the present invention, a "nucleic acid molecule specifically interfering with Kinesin gene expression" is a nucleic acid molecule which is able to reduce or to suppress the expression of gene coding for kinesin, in a specific way. It includes, but is not limited to siRNA, antisense and ribozyme molecules. The nucleic acid used according to the invention generally has a length of 15 to 50 nucleotides, preferably from 15 to 30 nucleotides in length.

In the present invention, the nucleic acid is capable of hybridizing specifically to a gene or transcripts coding for a kinesin, in particular KIF3A or KIF5B. Nevertheless, it is understood that the nucleic acid according to the invention does not need to have 100% complementarity with the target sequence to hybridize specifically. In particular, a nucleic acid with a degree of complementarity at least equal to approximately 90% is capable of hybridizing specifically. Preferably, the degree of complementarity between the nucleic acid according to the invention and the target sequence is equal to at least 95%, 96%, 97%, 98%, 99% or 100%. In a particular embodiment, the nucleic acid molecule specifically interfering with Kif3A gene expression comprises a sequence selected from the group consisting of siKIF3A_2, (GGUUCAGAAAGACAGGCAATT), siKIF3A_8 (GACCUGAUGUGGGAGUUUATT), and siKIF3A_9, (GCCUAAAGGAAGCUACAAATT).

The term "siRNA" or "interfering RNA" means any RNA which is capable of down-regulating the expression of the targeted protein. It encompasses small interfering RNA (siRNA), double-stranded RNA (dsRNA), single-stranded RNA (ssRNA), micro-RNA (miRNA), and short hairpin RNA (shRNA) molecules. RNA interference designates a phenomenon by which dsRNA specifically suppresses expression of a target gene at post-translational level. In normal conditions, RNA interference is initiated by double-stranded RNA molecules (dsRNA) of several thousands of base pair length. In vivo, dsRNA introduced into a cell is cleaved into a mixture of short dsRNA molecules called siRNA. The enzyme that catalyzes the cleavage, Dicer, is an endo-RNase that contains RNase III domains (Bernstein, Caudy et al. 2001 Nature. 2001 Jan 18;409(6818):363-6). In mammalian cells, the siRNAs produced by Dicer are 21-23 bp in length, with a 19 or 20 nucleotides duplex sequence, two-nucleotide 3' overhangs and 5'-triphosphate extremities (Zamore, Tuschl et al. Cell. 2000 Mar 31;101(1):25-33; Elbashir, Lendeckel et al. Genes Dev. 2001 Jan 15;15(2):188-200; Elbashir, Martinez et al. EMBO J. 2001 Dec 3;20(23):6877-88).

A number of patents and patent applications have described, in general terms, the use of siRNA molecules to inhibit gene expression, for example, WO 99/32619, US 20040053876, US 20040102408 and WO 2004/007718.

siRNA are usually designed against a region 50-100 nucleotides downstream the translation initiator codon, whereas 5'UTR (untranslated region) and 3'UTR are usually avoided. The chosen siRNA target sequence should be subjected to a BLAST search against EST database to ensure that the only desired gene is targeted. Various products are commercially available to aid in the preparation and use of siRNA.

In a preferred embodiment, the RNAi molecule is a siRNA of at least about 15-50 nucleotides in length, preferably about 20-30 base nucleotides, preferably about 20-25 nucleotides in length.

In a particular embodiment, the siRNA molecule comprises a sequence selected from the group consisting of siKIF3A_2 of SEQ ID No 1 (GGUUCAGAAAGACAGGCAATT), siKIF3A_8 of SEQ ID No 2 (GACCUGAUGUGGGAGUUUATT), and siKIF3A_9 of SEQ ID No 3 (GCCUAAAGGAAGCUACAAATT).

siRNA can comprise naturally occurring RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end of the molecule or to one or more internal nucleotides of the RNAi, including modifications that make the RNAi resistant to nuclease digestion.

RNAi may be administered in free (naked) form or by the use of delivery systems that enhance stability and/or targeting, e.g., liposomes, or incorporated into other vehicles, such as hydrogels, cyclodextrins, biodegradable nanocapsules, bioadhesive microspheres, or proteinaceous vectors (WO 00/53722), or in combination with a cationic peptide (US 2007275923). They may also be administered in the form of their precursors or encoding DNAs.

Antisense nucleic acid can also be used to down-regulate the expression of the kinesin. The antisense nucleic acid can be complementary to all or part of a sense nucleic acid encoding a kinesin e.g., complementary to the coding strand of a double-stranded cDNA molecule or complementary to an mRNA sequence, and it thought to interfere with the translation of the target mRNA

In a preferred embodiment, the antisense nucleic acid is a RNA molecule complementary to a target mRNA encoding a kinesin.

An antisense nucleic acid can be, for example, about 5, 10, 15, 20, 25, 30, 35, 40, 45 or 50 nucleotides in length. Particularly, antisense RNA molecules are usually 18-50 nucleotides in length.

An antisense nucleic acid for use in the method of the invention can be constructed using chemical synthesis and enzymatic ligation reactions using procedures known in the art. Particularly, antisense RNA can be chemically synthesized, produced by in vitro transcription from linear (e.g. PCR products) or circular templates (e.g., viral or non-viral vectors), or produced by in vivo transcription from viral or non-viral vectors.

Antisense nucleic acid may be modified to have enhanced stability, nuclease resistance, target specificity and improved pharmacological properties. For example, antisense nucleic acid may include modified nucleotides designed to increase the physical stability of the duplex formed between the antisense and sense nucleic acids, e.g., phosphorothioate derivatives and acridine substituted nucleotides.

Ribozyme molecules can also be used to decrease levels of functional kinesin. Ribozymes are catalytic RNA molecules with ribonuclease activity which are capable of cleaving a single-stranded nucleic acid, such as an mRNA, to which they have a complementary region. Thus, ribozymes can be used to catalytically cleave mRNA transcripts to thereby inhibit translation of the protein encoded by the mRNA. Ribozyme molecules specific for functional KIF3A or KIF5B can be designed, produced, and administered by methods commonly known to the art (see e.g., Fanning and Symonds, 2006, reviewing therapeutic use of hammerhead ribozymes and small hairpin RNA).

The interfering RNA, the antisense nucleic acids and the ribozyme molecule used according to the invention can be administered in the form of DNA precursors or molecules coding for them.

The kinesin inhibitors according to the invention are able, when they are introduced into infected macrophages, to induce decrease or suppression of the expression or activity of a kinesin, in particular to KIF3A or KIF5B, with a consequence being a significant decrease in the HIV production by infected macrophages.

### 1.3 Use of kinesin inhibitors of the invention.

The inventors showed that inhibition of KIF3A or KIF5B led to a strong reduction of HIV virions production by macrophages, and thus to a diminution of HIV viral load in the supernatant. The invention thus relates to a method for treating HIV infection by administering a therapeutically effective amount of a kinesin inhibitor, preferably a N-type kinesin inhibitor, more preferably a kinesin 1 and/or kinesin 2 inhibitor, even more preferably a Kif3A inhibitor and/or a KIF5B inhibitor, and to the use of such kinesin inhibitor for treating HIV infection in patients in need thereof. In particular, the treatment allows the improvement of the clinical outcome of a HIV-infected patient, through the diminution of HIV viral load.

The term "anti-HIV therapy" as used herein means any anti-HIV drug found useful for treating HIV infection in man alone, or as part of multidrug combination therapies, especially the triple and quadruple combination therapies called HAART. Typical suitable anti-HIV therapies include, but are not limited to multidrug combination therapies such as (i) at least those described in EP 1034790 A2.

The term "highly active antiretroviral therapy" or HAART as used herein means the multi-drug therapies used in current clinical treatment of HIV infections, including but not limited to the multi-drug therapies, e.g., the triple and quadruple drug therapies such as disclosed by A-M. Vandamme et al., in Antiviral Chemistry & Chemotherapy, 9: 187-203 (1998) which describes the current clinical treatments of HIV infections, including when to start multi-drug therapy and which drugs to combine. The triple drug therapy may include two nucleoside and nucleotide reverse transcriptase inhibitors ("NRTIs") and one protease inhibitor ("PI"), but there are many issues to be considered in the choice of the precise HAART for any patient. The highly active antiviral therapy (HAART) usually includes a combination of reverse transcriptase (RT) and protease inhibitors that induce undetectable levels of viral RNA in the peripheral blood plasma.

The term "nucleoside and nucleotide reverse transcriptase inhibitors" ("NRTI") as used herein means nucleosides and nucleotides and analogues thereof that inhibit the activity of HIV reverse transcriptase, the enzyme which catalyzes the conversion of viral genomic HIV RNA into proviral HIV DNA.

Typical suitable NRTIs include zidovudine (AZT) available under the RETROVIR tradename from Glaxo-Well-come Inc., Research Triangle(EP 1034790 A2)
The term "non-nucleoside reverse transcriptase inhibitors ("NNRTI"s)" as used herein means non-nucleosides that inhibit the activity of HIV reverse transcriptase.

Typical suitable non nucleoside reverse transcriptase inhibitors include nevirapine (EP 1034790 A2)

The term "protease inhibitor" ("PI") as used herein means inhibitors of the HIV protease, an enzyme required for the proteolytic cleavage of viral polyprotein precursors (e.g., viral GAG and GAG Pol polyproteins), into the individual functional proteins found in HIV infection. HIv protease inhibitors include compounds having a peptidomimetic structure, high molecular weight (7600 daltons) and substantial peptide character, e.g. CRIXIVAN (available from Merck) as well as nonpeptide protease inhibitors e.g., VIRACEPT (available from Agouron).

Typical suitable protease inhibitors. (EP 1034790 A2)

"HIV viral load" tests are reported as the number of HIV copies in a milliliter (copies/mL) of blood. If the viral load measurement is high, it indicates that HIV is reproducing and that the disease will likely progress faster than if the viral load is low. During treatment and monitoring, a high viral load can be anywhere from 5,000 to 10,000 copies/mL. Initial, untreated, and uncontrolled HIV viral loads can range as high as one million or more copies/mL. A low viral load is usually between 40 to 500 copies/mL, depending on the type of test used. This result indicates that HIV is not actively reproducing and that the risk of disease progression is low. A viral load result that reads "undetectable" does not mean that you are cured. It may mean that either the HIV RNA is not present in your blood at the time of testing or that the level of HIV RNA is below the threshold needed for detection. Even though HIV may be undetectable in the blood, it persists in cells and tissues throughout the body as "HIV provirus." HIV provirus refers to virus that has moved into cells and into the nucleus, where it has become integrated with the DNA of the host cell. This is also call "HIV proviral DNA" .Change in viral load is also a very important measurement. A rising count indicates either that the infection is getting worse or that you have developed resistance to the drugs that are being used for therapy, while a falling count indicates improvement and suppression of the HIV infection. Good clinical practice to minimize detectable HIV RNA plasma levels are known to those skilled in the art. See for example A-M. Vandamme et al., in Antiviral Chemistry & Chemotherapy, 9: 187-203 (1998) and "Drugs for HIV Infection" in The Medical Letter Vol.39 (Issue 1015) December 5, 1997, pages 111-116.

Compared to current anti-HIV treatments, one advantage of the use of a kinesin inhibitor according to the invention is its lesser vulnerability to virus mutation and subsequent escape from chemotherapy.

Accordingly, the present invention relates to
- a pharmaceutical composition comprising a kinesin inhibitor, and optionally a pharmaceutically acceptable carrier, in particular for use in the treatment of HIV infection, optionally in combination with another anti-viral treatment;
- a kinesin inhibitor, and optionally a pharmaceutically acceptable carrier, for use in the treatment of HIV infection, optionally in combination another anti-viral treatment;
- the use of a kinesin inhibitor for the manufacture of a medicament for the treatment of HIV infection, optionally in combination with another anti-viral treatment;
- a method for treating HIV infection in a subject in need thereof, comprising administering an effective amount of a pharmaceutical composition comprising a kinesin inhibitor and optionally a pharmaceutically acceptable carrier;
- a combined preparation, product or kit containing (a) a kinesin inhibitor and (b) an anti-viral treatment as a combined preparation for simultaneous, separate or sequential use, in particular in the treatment of HIV infection; and
- a method for treating HIV infection in a subject in need thereof, comprising administering an effective amount of a pharmaceutical composition comprising a kinesin inhibitor, and an effective amount of a pharmaceutical composition comprising an anti-viral agent.

In one aspect, the present invention relates to a pharmaceutical composition comprising at least one kinesin inhibitor according to the invention optionally with a pharmaceutically acceptable carrier.

In one embodiment, said inhibitor is a N-kinesin inhibitor. More preferably, said kinesin inhibitor is a kinesin 2 and/or a kinesin 1 inhibitor. Even more preferably, said inhibitor is a Kif3A or KIF5B inhibitor.

Preferably, said inhibitor is a N-kinesin selective inhibitor. More preferably, said kinesin is a kinesin 2 selective inhibitor. Even more preferably, said inhibitor is a Kif3A selective inhibitor, a KIF5B selective inhibitor or an inhibitor selective for both KIF3A and KIF5B. According to a preferred embodiment, said inhibitor is a nucleic acid comprising or consisting of a sequence able to hybridise with a gene or mRNA coding for a kinesin, in particular KIF3A or KIF5B, and to decrease or suppress the expression of said kinesin. The kinesin inhibitor used in the pharmaceutical composition of the invention is present in a therapeutically effective amount. The term "therapeutically effective amount" as used in the present application is intended an amount of therapeutic agent, a kinesin inhibitor, administered to a patient that is sufficient to constitute a treatment of HIV infection as defined above.

The pharmaceutical composition comprising the kinesin inhibitor is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

Possible pharmaceutical compositions include those suitable for oral, rectal, intravaginal, mucosal, topical (including transdermal, buccal and sublingual), or parenteral (including subcutaneous, intramuscular, intravenous and intradermal) administration. For these formulations, conventional excipient can be used according to techniques well known by those skilled in the art.

The compositions for parenteral administration are generally physiologically compatible sterile solutions or suspensions which can optionally be prepared immediately before use from solid or lyophilized form. Adjuvants such as a local anesthetic, preservative and buffering agents can be dissolved in the vehicle and a surfactant or wetting agent can be included in the composition to facilitate uniform distribution of the active ingredient.

For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. Pharmaceutical compositions according to the invention may be formulated to release the active drug substantially immediately upon administration or at any predetermined time or time period after administration.

Pharmaceutical compositions according to the invention can comprise one or more kinesin inhibitor(s) associated with pharmaceutically acceptable excipients and/or carriers. These excipients and/or carriers are chosen according to the form of administration as described above. Other active compounds can also be associated with kinesin inhibitors, in particular antiviral drugs such as the ones used in anti-HIV therapy, preferably used in HAART therapy including the ones disclosed above. The initiation of the HAART may occur before, after or concurrently with administering a therapeutically effective amount of a kinesin inhibitor according to the invention, preferably a N-type kinesin inhibitor, more preferably a kinesin 1 and/or kinesin 2 inhibitor, even more preferably a KIF3A or KIF5B inhibitor in accordance with the present invention.

Such combination with other antiviral treatments is aimed at reinforcing the desired effects. Indeed, adding a kinesin inhibitor to such an anti-HIV therapy allowing a significant decrease of the viral load should allow to clear viral reservoirs in macrophages, with the aim of eradicating the virus.

The amount of kinesin inhibitor to be administered has to be determined by standard procedure well known by those of ordinary skill in the art. Physiological data of the patient (e.g. age, size, and weight), the routes of administration and the disease to be treated have to be taken into account to determine the appropriate dosage.

The kinesin inhibitor may be administered as a single dose or in multiple doses. If the kinesin inhibitor is a small molecule inhibiting the kinesin activity, each unit dosage may contain, for example, from 200 to 1000 mg/kg of body weight, particularly from 500 to 800 mg/kg of body weight. If the kinesin inhibitor is an anti-kinesin antibody, each unit dosage may contain, for example, from 0.1 to 20 mg/kg of body weight, particularly from 4 to 10 mg/kg of body weight. If the kinesin inhibitor is a kinesin RNAi molecule, each unit dosage may contain, for example, from 2 to 50 mg/kg of body weight, particularly from 5 to 20 mg/kg of body weight. If the kinesin inhibitor is a kinesin dominant negative receptor such as a cargo-only kinesin domain, each unit dosage may contain, for example, from 5 to 100 mg/kg of body weight, particularly from 15 to 70 mg/kg of body weight.

### 2. Tagged kinesin

It has been up to now difficult to characterize more precisely the intracellular trafficking and targeting of kinesin intracytoplasmic protein. Therefore, one aspect of the present invention is to provide new tools allowing study of the intracellular trafficking of kinesins, identification of intracellular partners as well as identification and screening of molecules able to inhibit such intracellular trafficking.

Kinesins have often been tagged by replacing the cargo domain with a fluorescent protein (Douglas S. Martin et al, PNAS 2010 March 23; 107(12): 5453-5458; Jennetta W Hammond et al, PLoS Biol.2009 March; 7(3)). However, such constructions are not functional as they do not transport anymore their cargo protein and therefore prevents any definitive conclusions on the role of such cargo protein when transported along microtubules.

To further elucidate the role of KIF3A in HIV infection, the inventors investigated the dynamics of KIF3A in infected macrophages using an original construct where the fluorescent protein mCherry has been inserted between the stalk and the cargo domain of Kif3A, flanked by two spacers derived from antibody fragment to provide flexibility. This KIF3A-internal mCherry construct (KIF3A-iCh) was readily well expressed as seen by western blot and immunofluorescence using an antibody specific for the 20 last amino acids of KIF3A (i.e. within the cargo domain). The fusion Kif3A-iCh protein was observed to move on microtubules (identified by co-expression of EGFP-α-tubulin).

Primary macrophages were then co-infected with HIV Gag-iGFPΔEnv virus and KIF3A-iCh lentiviral vector. Thus, the inventors were able to show that KIF3A was colocalized with Gag+ compartments-containing virions, using such tagged Kif3A-iCh kinesin.

The inventors have indeed unexpectedly showed that a tag protein such as the fluorescent protein mCherry, inserted between the stalk and the cargo domain of KIF3A kinesin protein, did not impair the cargo domain activity and enabled to follow the location or targeting of the protein inside the cell.

The present invention thus features tagged kinesin proteins which have a variety of different uses including being used as a tool to study kinesin function, transport of its cargo proteins, and identification of intracellular partners. In particular, such tagged kinesin can be used to study the dynamics of viral infection, in particular HIV infection, as well as to screen for kinesin inhibitors. Such tagged kinesins according to the invention are able to maintain the functional activity of a kinesin as above defined and in particular are able to move cargo proteins along microtubules. In addition, they can be easily detected using their tag portion.

Therefore, the invention relates to the use, particularly *in vitro* or *ex vivo*, of a tagged kinesin according to the invention, preferably a N-type tagged kinesin, more preferably a tagged kinesin 1 or a tagged kinesin 2, even more preferably KIF3A or KIF5B, to screen for kinesin inhibitors. Of particular interest is the use of a tagged KIF3A kinesin to screen for KIF3A kinesin inhibitors useful in the treatment of HIV infection.

### Recombinant hybrid kinesin polypeptide

The invention thus relates to a tagged kinesin comprising a kinesin motor domain, a stalk domain, a cargo domain and a tag allowing said tagged kinesin to be detected, and wherein said tag is located between said stalk domain and said cargo domain. In a preferred embodiment, said tag is flanked at least on one side, preferably on both sides, by spacers providing flexibility.

In one embodiment, said tagged kinesin is a chimeric kinesin, wherein at least one of the 3 major domains (motor, stalk and cargo) belong to a native kinesin different from the kinesin from which at least one of the other 2 domains are derived. For example, such a chimeric kinesin may comprise a kinesin 2 motor domain, (e.g. amino acid sequence comprised between amino acids N°s 1-350 of SEQ ID N°4), whereas the cargo and/or stalk domain belong to a kinesin 1.

In another embodiment, the tagged protein of the invention comprises a motor domain, a stalk domain and a cargo domain all belonging to the same native kinesin, preferably to a N-type kinesin, more preferably to a kinesin 1 or to a kinesin 2, even more preferably to KIF3A or KIF5B. In a further preferred embodiment, said motor domain comprises an amino acid sequence at least 75, 80, 85, 90, 95, or 98 % identical to the amino acid sequence comprised between amino acids 1-350 of SEQ ID N°4, said stalk domain comprises an amino acid sequence at least 75, 80, 85, 90, 95, or 98 % identical to the amino acid sequence comprised between amino acids 351-594 of SEQ ID N°4 and cargo domain comprises an amino acid sequence at least 75, 80, 85, 90, 95, or 98 % identical to the amino acid sequence comprised between amino acids 862-968 of SEQ ID N°4. Therefore, the invention particularly relate to a tagged kinesin comprising a KIF3A motor domain, preferably comprising an amino acid sequence at least 75, 80, 85, 90, 95, or 98 % identical to the amino acid sequence comprised between amino acids 1-350 of SEQ ID N°4, a KIF3A stalk domain, preferably comprising an amino acid sequence at least 75, 80, 85, 90, 95, or 98 % identical to the amino acid sequence comprised between amino acids 351-594 of SEQ ID N°4, a KIF3A cargo domain preferably comprising an amino acid sequence at least 75, 80, 85, 90, 95, or 98 % identical to the amino acid sequence comprised between amino acids 862-968 of SEQ ID N°4, and a tag allowing said tagged kinesin to be detected, and wherein said tag, preferably a fluorescent tag protein, more preferably a mCherry protein, is located between said KIF3A stalk domain and said KIF3A cargo domain protein.

Within the scope of the invention are kinesin motor domains, kinesin stalk domains and kinesin cargo domains derived from naturally-occurring kinesin proteins as well as those comprising amino acid changes (addition, deletion, mutation) that do not affect significantly the functional activity of said kinesin.

### Tag protein

The tagged kinesin according to the invention comprises a so-called "tag" peptide, polypeptide or protein which could advantageous be used for detection and localisation of said tagged kinesin. Any type of tag known to those skilled in the art may be used provided it does not affect significantly the kinesin activity including BCCP-tag, c-myc-tag, calmoduline-tag, FLAG-tag, HA-tag, His-tag, Maltose binding protein-tag, Nus-tag, Glutathione-S-transferase-tag, Green fluorescent protein-tag, Thioredoxin-tag, S-tag, Strep-tag, human protein C tag, Chitin binding protein tag, T7-tag, Myc-tag, V5-tag, VSV-tag, Avi.tag, BioEase-tag, SNAP-tag, FlaSH-tag, Nus A-tag, DsbA-tag , GFP, EGFP, mCherry, Emerald, Topaz, W1b. (EGFP, Emerald , Topaz, and W1b are derivatives of GFP.)

In a preferred embodiment said tag is a fluorescent peptide or protein such the Green Fluorescent Protein (GFP) and the red fluorescent protein mCherry, more preferably selected from the group consisting of GFP-tag, EGFP, mCherry, Emerald, Topaz, W1b, and their fluorescent derivatives.

A "fluorescent protein" region contains a chromophore that fluoresces. Preferably, the fluorescent protein region is the red cherry fluorescent protein. Preferred derivatives have a sequence similarity of at least about 75%, at least about 85%, or at least about 95% to the cherry protein.

Said tag can be of any length compatible with its function as a tag, generally between 5 and 100 amino acids.

### Spacer sequence

Additionally, the tagged kinesin according to the invention may contain a spacer peptide or polypeptide. Indeed, the tag polypeptide region is linked either directly, i.e. contiguously, to the stalk domain on one side and to the cargo domain on the other side, or indirectly, i.e. through a polypeptide linker, to at least one of the kinesin stalk or cargo domain. Tagged kinesin fusion proteins contain a kinesin polypeptide region and a fluorescent protein region either directly joined together or joined together through a linker. In one embodiment, said tag, preferably a fluorescent tag, is flanked by spacers at both ends. Such spacers (also called linkers) provide flexibility to the overall construct, allowing the tagged kinesin to still function as a kinesin while being able to be detected through its tag portion.

Such spacers are known to those skilled in the art. They usually comprise, or consist essentially of non polar amino acids such as Glycine, Serine or Alanine residues. Some of them derive from immunoglobulin amino acid sequences. In one specific embodiment, said spacers comprise or consists in a series of reiterated glycine and/or alanine residues, such as repetitions of Gly-Gly-Gly-Gly-Ser peptide or an analogous derived sequence.

In a preferred embodiment, the invention relates to a tagged kinesin comprising a motor domain, a stalk domain, a cargo domain and a tag, preferably a fluorescent tag, even more preferably mCherry, allowing said tagged kinesin to be detected, wherein said tag is located between said stalk domain and said cargo domain, and is flanked by spacers, preferably by spacers comprising or consisting in chains of GGGGS residues. Said tagged kinesin is preferably a N-type kinesin, more preferably a kinesin 1 or kinesin 2, even more preferably KIF3A or KIF5B. In on embodiment, said kinesin comprises the amino acid sequence of SEQ ID N°4.

The various polypeptides of the present invention can be prepared in any suitable manner including isolation from natural sources, recombinantly produced polypeptides, synthetically produced polypeptides, or polypeptides produced by a combination of these methods.

### Nucleic acid comprising tag

The present invention also relates to a nucleotide sequence coding for a tagged kinesin such as defined above.

Another aspect of the present invention describes a nucleic acid encoding a tagged kinesin protein according to the invention. Such nucleic acid comprises either a contiguous nucleotide sequence that codes for the protein or a sequence that is processed by a host cell to produce a contiguous nucleotide sequence encoding for the protein. Another aspect of the present invention describes an expression vector comprising a nucleic acid encoding a tagged kinesin protein described herein. Nucleic acids able to encode any of the tagged kinesins described in the present specification are contemplated. In an embodiment, said nucleic acid comprises the amino acid sequences of a tagged KIF3A or KIF5B, particularly the amino acid sequence of SEQ ID N°5.

### Vector

The present invention also relates to a vector, particularly a plasmid, containing a nucleotide sequence such as defined above. In particular, the invention relates to an expression vector comprising a nucleic acid sequence encoding a tagged kinesin according to the invention.

The term "vector" refers to DNA molecule used as a vehicle to transfer recombinant genetic material into a host cell. The four major types of vectors are plasmids, bacteriophages and other viruses, cosmids, and artificial chromosomes. The vector itself is generally a DNA sequence that consists of an insert (a heterologous nucleic acid sequence, transgene) and a larger sequence that serves as the "backbone" of the vector. The purpose of a vector which transfers genetic information to the host is typically to isolate, multiply, or express the insert in the target cell. Vectors called expression vectors (expression constructs) are specifically adapted for the expression of the heterologous sequences in the target cell, and generally have a promoter sequence that drives expression of the heterologous sequences.

Generally, the regulatory elements that are present in an expression vector include a transcriptional promoter, a ribosome binding site, a terminator, and optionally present operator. Another preferred element is a polyadenylation signal providing for processing in eukaryotic cells. Preferably, an expression vector also contains an origin of replication for autonomous replication in a host cell, a selectable marker, a limited number of useful restriction enzyme sites, and a potential for high copy number.

An expression vector contains recombinant nucleic acid encoding for a polypeptide along with regulatory elements for proper transcription and processing. The recombinant nucleic acid contains two or more nucleic acid regions not naturally associated with each other. Exogenous regulatory elements such as an exogenous promoter can be useful for expressing recombinant nucleic acid in a particular host. Examples of expression vectors are cloning vectors, modified cloning vectors, specifically designed plasmids and viruses. Expression vectors providing suitable levels of polypeptide expression in different hosts are well known in the art. Mammalian expression vectors well known in the art include pcDNA3 (Invitrogen), pMC1neo (Stratagene), pXT1 (Stratagene). Bacterial expression vectors well known in the art include pET11a (Novagen), lamda gt11 (Invitrogen). Fungal cell expression vectors well known in the art include pYES2 (Invitrogen) and Pichia expression vector (Invitrogen).

Examples of techniques for introducing nucleic acid into a cell and expressing the nucleic acid to produce protein are provided in references such as Ausubel, Current Protocols in molecular biology, John wiley, 1987-1998, and Sambrook, et al., in Molecular cloning, A laboratory Manual 2nd Edition, Cold Spring Harbor Laboratory Press, 1989.

Expression vectors may be introduced into host cells using standard techniques. Examples of such techniques include transformation, transfection, lipotransfection, protoplast fusion, and electroporation.

### Host cell

The present invention relates to a host cell chosen among any type of cells, particularly human cells, transformed with a vector such as defined above. Of particular interest are macrophages and monocytes, either primary cells or cell lines, transformed with an expression vector encoding a tagged kinesin according to the invention.

Another aspect of the present invention describes a host cell comprising a nucleic acid encoding a tagged kinesin fusion protein, particularly an expression vector encoding a tagged kinesin according to the invention. The nucleic acid may be part of the host genome or may exist independently of the host genome.

Another aspect of the present invention describes a non-human transgenic animal comprising such a nucleic acid encoding a tagged kinesin protein.

### Method for expressing a recombinant polypeptide

Tagged kinesin proteins can be produced using techniques well known in the art. Preferably, such proteins are produced by recombinant expression inside a host cell by way of an expression vector or by way of nucleic acid integrated into the host genome. Typically, natural isolated nucleic acid encoding the mCherry proteins and the KIF3A kinesin protein are spliced into expression construct using conventional methodologies, see e.g Molecular cloning, a laboratory Manual (Sambrook, et al. Cold Spring Harbor Laboratory), current protocols in Molecular Biology (Eds. Ausubel, et al., Greene Publ. Assoc., Wiley-Interscience, NY). Alternatively, the amino acid sequences of the subject peptides are used to back-translate peptide-encoding nucleic acids optimized for selected expression systems (Holler et al. (1993) Gene 136, 323-328; Martin et al. (1995) Gene 154, 150-166). In either instance, the constructs are designed for expression in any conventional system, such as bacteria or eukaryotic cells.

### 3. Screening of kinesin inhibitors for their use against HIV infection.

The present invention also concerns a method of screening of molecules useful in the treatment of HIV infection based on the ability of such molecules to inhibit the expression and/or activity of a kinesin of interest, preferably N-type kinesins, more preferably kinesin-1 or kinesins 2, even more preferably KIF3A or KIF5B. It relies on the inventors' observations that the host cell KIF3A kinesin protein pathway influences retroviral infection.

The recombinant protein, described above could be used to analyse the recruitment of different host proteins necessary for at least its transport to the virions formation, and the formation of the viral particles and/or to screen for molecules able to inhibit the transport of various cargo virus proteins, in particular HIV proteins along microtubules. As indicated here above, several kinesin inhibitors are already known to the skilled in the art. However, it may be desirable to isolate new kinesin inhibitors being suitable for the treatment of HIV infection.

### 3.1 Methods of screening

In one embodiment, the present invention is a method of screening test agents as inhibitors of HIV virions formation comprising the step of determining whether the test agent is a kinesin, preferably a kinesin-1 or a kinesin-2, even more preferably a KIF3A or KIF5B kinesin inhibitors. If the test agent is a KIF3A or KIF5B inhibitor, then the agent is a suitable inhibitor of HIV virions formation. In a preferred embodiment, the present invention is a method of screening test chemical or compounds for inhibition of HIV infection, comprising the steps of exposing the test chemical or compound to a cell, and determining whether said test compound inhibits the gene expression or activity of a kinesin, preferably a kinesin-2 and even more preferably kinesin KIF3A or KIF5B, as measured by the HIV virions release in the supernatant or localisation of known KIF3A or KIF5B fluorescent protein partners.

In another embodiment, the present invention is a method of screening test chemicals or compounds for inhibition of HIV virions secretion. In one embodiment of the first aspect, the method comprises the steps of (a) primary cells infection, (b) exposing a test chemical or compound to the primary infected cells, (c) examining the results of step (a) and (b). The present invention is thus a method of screening test agents as inhibitors of HIV new virions formation. If the test agent is a kinesin activity or expression inhibitors, then the agent is a suitable inhibitor of HIV infection, in particular in HIV new virions formation. In a second embodiment, the present invention is a method of screening test agent as inhibitors of kinesin expression or activity. If the test agent inhibits the localisation of a known KIF3A fluorescent protein partners at the cell periphery, then the agent is a suitable inhibitor of HIV infection, in particular in HIV new virions formation.

### 3.1.1 Test agents, chemicals, or compounds

One embodiment of the present invention relates to a screen for kinesin inhibitors, preferably kinesin 2 inhibitors, even preferably KIF3A or KIF5B kinesin inhibitors. The identified inhibitors are expected to inhibit a step coincident with virus establishment of HIV in human cells and inhibit subsequent HIV virions formation.

One of skill in the art would understand that many different chemicals or compounds could be screened for inhibition of kinesin, preferably kinesin 1 or 2, even preferably KIF3A or KIF5B, including small molecules, natural products, peptides, and proteins. Also included would be nucleic acids, such as siRNAs, small hairpin RNAs (shRNAs), antisense oligonucleotides, and ribozymes.

Suitable groups of compounds would include the Hembridge collection (16000 compounds), ChemDiv collection (20000 compounds), and the NCl open collection (140000 compounds). All of the terms indicate a test substance that one would evaluate as a screen for HIV inhibition.

### 3.1.2 Cell lines

In a preferred embodiment one would use macrophage primary cells. One of skill in the art would understand that other human and mammalian cells could replace macrophage primary cells. Any mammalian cell line that is able to be infected by HIV would be useful, especially monocytes/macrophages cell lines such as U937.

### 3.1.3 Screening

In particular, the invention is drawn to a method for screening for compounds useful for the treatment of HIV infection comprising the steps of:
a) providing or obtaining a candidate compound; and
b) determining whether said candidate compound inhibits the activity and/or expression of a kinesin, preferably a N-type kinesin, more preferably a kinesin 1 or a kinesin 2, even more preferably kinesin KIF3A or KIF5B,
wherein the ability of said candidate compound to inhibit the expression or activity of said kinesin indicates that said candidate compound is indicative of its usefulness for the treatment of HIV infection.

The candidate compound to be tested in the frame of this method may be of any molecular nature, for example it may correspond to a chemical molecule (preferably a small molecule), an antibody, a peptide, a polypeptide, an aptamer, a siRNA, a sense or antisense oligonucleotide, or a ribozyme.

The ability of said candidate compound to inhibit the expression or activity of a kinesin of interest may be tested using any of the methods known to those skilled in the art, including those mentioned in the present specification, for example those mentioned in the section entitled "1. Kinesin inhibitors as therapeutic agents in HIV infection".

In one embodiment of such screening method, the chimeric kinesin described in the above section might be used. In particular, to screen for kinesin inhibitors, the original construct where the fluorescent protein mCherry has been inserted between the stalk and the cargo domains of native Kif3A or KIF5B, flanked by two spacers derived from antibody fragment to provide flexibility may be used. Therefore, in one embodiment of the screening method of the invention, step b) of said method consists in determining whether said candidate compound inhibits the activity of a tagged kinesin preferably a N-type kinesin, more preferably a kinesin 2, even more preferably a KIF3A or KIF5B according to the invention. In a further embodiment, said step b) consists in determining whether said candidate compound inhibits the binding of said tagged kinesin to a cargo protein.

The screening method according to the invention may further comprise, for such candidate compound having the ability to inhibit the expression and/or activity of a kinesin of interest, the step of determining whether said candidate compound is able to inhibit and/or decrease HIV infection. In one embodiment, such step c) comprises administering said candidate compound to a cellular model or to a non-human animal model of a HIV infection in order to confirm that the candidate compound is suitable for the treatment of HIV infection. In a further embodiment, said step c) comprises determining whether said candidate compound interferes with HIV virions formation, preferably using primary macrophages/monocytes or macrophage/monocyte cell lines infected with HIV, more preferably of human origin.

In another embodiment, step c comprises determining whether said candidate compound has an impact on viral load.

Furthermore the present invention also comprises a method of screening for candidate substances for their ability to modulate viral load using a fluorescent KIF3A-iCherry protein.

In one embodiment, the invention therefore relates to a method of screening for candidate compounds able to modulate viral load, comprising the steps of
a) contacting said candidate compound with a cell infected with a retrovirus, preferably HIV, wherein said candidate compound is a kinesin inhibitor,
b) measuring viral load in said cell, and
c) determining whether said kinesin inhibitor candidate decrease said viral load.

In one embodiment, said method comprises a first step prior to step a) of determining whether said candidate compound is a kinesin inhibitor, preferably a kinesin-2 or a kinesine-1 inhibitor, even preferably KIF3A or KIF5B.

The following examples are given for purposes of illustration and not by way of limitation.

### Examples

### Materials et Methods

### Plasmids, Antibodies and Reagents

pBR NL4-3 Gag-iGFP T-tropic envelope was a gift from B. Chen²⁹. Original envelope has been replaced by th_174 V3 loop macrophage-tropic envelope. pENTR KIF3A (NM_007054.1) has been purchase from Genecopoeia. To construct KIF3A-iCherry, the cDNA for the mCherry fluorescent protein, flanked by linkers (amino acid sequence GGGGSGGGGSGGGGS), has been inserted between the stalk and the cargo domain region of KIF3A. The resulting cDNA KIF3A-iCherry has been cloned into a pExp plasmid using Gateway technology.

Antibodies specific for KIF3A (Abcam), CD81 (Abcam), Gag p24 (KC57-FITC, Beckman Coulter), Gag p24 (Abcam), α-Tubulin (Serotec) were used according to the manufacturers' recommendations. Antibodies specific for p17 (NIH4811) and gp120 (NIH1476) were provided by the NIH AIDS Reagent Program. Secondary antibodies conjugated with Alexa Fluor 488 (Invitrogen), Cy3 or Cy5 (Jackson) were used according to the manufacturers' instructions. Nocodazole (Sigma) was dissolved in DMSO at a 10mM stock concentration. M-CSF (Calbiochem) was dissolved in ultrapure water at a 25 µM stock.

### Primary Cells and Cell Lines

PBMC were purified from blood from healthy donors and monocytes were isolated by positive selection using CD14+ Microbeads from Miltenyi. Monocytes were differentiated for 7 days in macrophages by culture on non treated culture plastic (Nunc) in RPMI 1640 supplemented with 10% FCS (Gibco) and M-CSF (25 mg/ml final). Ghost, HEK293T, TZM-B1 and HeLa cells were cultured in DMEM glutamax whereas Jurkat T cells were cultured in RPMI 1640. Both mediums were supplemented with 10% FCS (Gibco).

### Virus Preparations and Infections

The following virus strains have been produced and used in the present study: NL4-3 AD8 (NLAD8), NL4-3 ΔEnv, HIV Gag-iGFP, HIV Gag-iGFP ΔEnv. Viruses were produced by transfection of the corresponding proviral cDNA in 293T cells by calcium phosphate/DNA co-precipitation. Pseudotyping was eventually achieved by co-transfection of the plasmid encoding the VSV-G envelope protein. Supernatant were harvested 48-72 hr after transfection, and ultracentrifuged at 100,000g for 90 min. Pellets were resuspended in 2 % BSA in PBS. Virus preparations were titrated by infecting the Ghost reporter cell line and infectivity was measured 24 hr post-infection by flow cytometry.

NLAD8-infected macrophage samples were prepared for immuno-electron microscopy as previously described ¹². For the other infection experiments, macrophages were infected at a Multiplicity of Infection (MOI) of 2 and kept in culture for indicated times.

### Viral production and Infectivity assays

Viral release was monitored in supernatants by p24 ELISA (Ingen). Virion-containing supernatants were normalized for equal amounts of p24 and infectious titers were determined using TZM-bl indicator cells as described ³⁰. Briefly, the volume of supernatant equivalent to 2 ng of p24 was incubated with 10.000 TZM-bl cells for 24 hr and assay was developed using the GalScreen kit (Applied Biosystems) for chemo luminescent detection of reporter β-galactosidase.

### RNA interference

The sequences of the various siRNA used in the present study are given in Table 1. Macrophages were transfected using Interferin (Polyplus). Briefly, siRNA (100nM final) were diluted in 250 µl of OptiMEM (Gibco) mixed with 5 µl of Interferin and left at room temperature for 15 minutes. Complexes were added drop-wise onto the macrophage culture. Cells were assayed 3 days later.

### Western blotting

Cells were directly lysed into the well in 0.5% NP40 lysis buffer for 30min at 4°C. Cell lysates were analyzed on 4-12% Bis-Tris Acrylamid gels (NuPAGE, Invitrogen) followed by immunoblotting.

### Immunofluorescence

Cells were fixed in methanol at -20°C for 5 sec and incubated in 0.2 % BSA in PBS. Antibody staining and washes were performed in the same buffer, before mounting coverslips in moviol. Samples were imaged on a Nikon Ti Inverted Microscope fitted with a confocal A1R system. Using a 60x oil immersion objective with a numerical aperture of 1.4, confocal images were collected as a 3D stack with a focal step size from 0.25 to 0.5 µm.

### Live imaging

Long time-lapse movies (days) were acquired using a Nikon Biostation system using 20x and 40x dry objective. Fast imaging was performed on a Nikon Ti Inverted Microscope fitted with a video-rate confocal system consisting of a spinning disk confocal head (Yokogawa). Using a 100x oil immersion objective with a numerical aperture of 1.4 and confocal images were collected with a HQ2 camera.

### Correlative light-electron microscopy

Purified monocytes were plated with M-CSF on CELLocate coverslips (eppendorf) that etched grids with coordinates, allowing the cell of interest to be found through all of the steps of the procedure. After 7 days, macrophages were co-infected with HIV-1 Gag-iGFP V3_loop and KIF3A-iCherry lentiviral vector. At day 7 post-infection, cells were fixed in 2.5% glutaraldehyde in 0.2M Cacodylate buffer and imaged with a spinning disk confocal microscope. Then, coverslips were embedded in epon and processed for electron microscopy¹².

### Image Analysis

Micrographs were processed with either NIS Element, MetaMorph, Imaris or ImageJ software according to the kind of analysis to be performed.

**Table 1. Name and target sequences of the siRNA used**

| Name | Target sequence |
|---|---|
| KIF3A_2 | GGUUCAGAAAGACAGGCAA |
| KIF3A_8 | GACCUGAUGUGGGAGUUUA |
| KIF3A 9 | GCCUAAAGGAAGCUACAAA |
| Luc | CGUACGCGGAAUACUUCGA |
| KIF4A | CAAUUGAUUACCCAGUUAU |
| Tsg101 | CCUCCAGUCUUCUCUCGUC |

TT DNA modifications have been added at the 3' end of all siRNA sequences, which were purchased from Eurogentec. KIF4A and Tsg101 siRNA have been previously shown to efficiently knockdown their targets (Martinez NW et al, 2008; Garrus JE et al, 2001).

### Example 1 Intracellular expression and localization of Gag in macrophages following their infection by HIV-1

Given that Gag is the maestro of the viral assembly process, we wanted to first establish its precise distribution as a function of time in HIV-1-infected macrophages. Throughout this study we used primary macrophages derived from monocytes purified from blood of healthy donors and in vitro-infected with VSV-G pseudotyped HIV-1 NLAD8, unless specified otherwise.

First, three dimensional reconstruction of Gag staining of macrophages at 7 days post infection (p.i.) highlighted the diversity of Gag distribution (Figure 1A), which encompassed cytosolic diffuse staining, as well as large internal compartments scattered in the central area of the cells. Such large compartments contained Env and CD81 (Figure 1B), and also CD9 and Tetherin (not shown), which identified them as viral assembly compartments where viruses are produced and stored in primary macrophages^{8,10,11,13,21}. Investigating the kinetics of Gag expression and localization in HIV-infected macrophages, we observed at day 2 p.i. that Gag appeared only as a diffuse staining in the cytosol of the infected cells (Figure 1C, upper panel). At day 7 p.i., Gag staining remained in the cytosol but was also concentrated in intracellular compartments of various sizes in the µm range (Figure 1C, lower panel). Usage of a p24Gag specific antibody which recognizes the Capsid only when cleaved from the precursor, identified compartments containing virions present at day 7 but not at day 2 p.i..

To further establish the temporal order of events, we derived a HIV Gag-iGFP virus coding for a fusion protein where the GFP has been inserted between the Matrix and the Capsid domains of Gag, flanked by protease sites (see details of the construction of the virus in supplemental information and its characterization Figure S1). Gag intracellular distribution was similar in macrophages infected with HIV Gag-iGFP or with wild type HIV with respect to CD81 and Env, which co-localized together with Gag in both conditions (Figure 1B). Thus HIV Gag-iGFP shared essential features of HIV wild type and was suitable for kinetics studies in macrophages. Time-lapse microscopy of primary macrophages infected with HIV Gag-iGFP was recorded for 5 days. Analysis of movies recorded for up to 7 days p.i. clearly confirmed our data. Gag-iGFP started to be visible in the cytosol within 2 to 3 days p.i.; Gag+ compartments appeared 1 day later and then accumulated in the central area of the cells while the cells started to project membrane extensions in multiple directions (Figure 1D). Thus, following infection of primary macrophages by HIV-1 in vitro, Gag was first expressed in the cytosol and then concentrated in internal compartments.

To distinguish viral particles containing Gag from unassembled Gag, we analyzed Gag distribution as well as the one of Env at the ultrastructural level by immuno-electron microscopy (immuno-EM) (Figure 1E). Newly formed viral particles were present either as nascent buds (arrowheads), or as immature or mature viral particles in the lumen of the compartments. Interestingly, Gag was also present in the cytosol in the vicinity of the compartments whereas Env was only found associated with viral particles. We noticed the presence of a thick molecular coat at the limiting membrane of the compartments which was typical of viral assembly compartments in macrophages ¹⁰⁻¹² and which nature remains elusive.

Therefore, Gag, which is known to be synthesized on free polysomes throughout the cytosol, has to reach precise intracellular sites where assembly will take place.

### Example 2 Microtubule-dependent distribution of virus-containing compartments

Macrophages possess a large cytosolic volume suggesting that Gag site of synthesis can be relatively far from the site of viral assembly. We evaluated the potential role of the microtubules in the transport of Gag looking at the distribution of Gag and tubulin in HIV-1-infected macrophages. Examination by confocal microscopy of HIV-1-infected macrophages mock-treated with DMSO revealed a very rich tubulin network (Figure 2A). Large Gag+ compartments were present and usually scattered in the central area of the cells. Given the density of the tubulin network it was difficult to evaluate at this level of resolution whether these compartments were tightly associated with the network.

If Gag and Gag+ compartments are transported along microtubules, then disruption of the microtubule network should dramatically affect Gag distribution. Treatment of HIV-1-infected macrophages for 1 hr with Nocodazole disrupted the microtubule network and only a few nocodazole-resistant tubules remained (Figure 2A). Strikingly, all Gag+ compartments concentrated in the perinuclear area. Conversely, most of the cytosolic Gag staining outside of the large compartments disappeared. Moreover, upon nocodazole washout, we observed within 15 min that the Gag+ compartments again became spatially dispersed (Figure S2), supporting the idea that movements of Gag+ compartments were directed by microtubules.

Given the diversity of the Gag distribution among HIV-infected macrophages ⁹, we wanted to image the very same cells before and after exposure to nocodazole. Living macrophages infected with HIV Gag-iGFP virus were recorded by time-lapse microscopy just before and after drug addition for 30 min. Indeed, we observed with time the nocodazole-induced redistribution of Gag-iGFP+ structures towards one pole of the cell nucleus (Figure 2B). Thus, localization of Gag+ compartments was dependent of the integrity of the microtubule network.

To get further insights into the relationship between Gag+ compartments and tubulin, we performed immuno-EM, the only technique allowing unambiguously to distinguish Gag+ viral particles from Gag protein. Ultrathin cryosections of HIV-1-infected macrophages were labeled with antibodies specific for Gag and tubulin. At the ultrastructural level, α-tubulin specific labeling was rich and nicely decorated microtubules in HIV-1-infected macrophages (Figure 2C). Looking at the viral assembly compartments identified through Gag labeling of particles, microtubules were present in the vicinity but mostly absent from the viral particles (Figure 2C). Strikingly most of the virus containing compartments had at least one bundle of microtubules adjacent to the limiting membrane of the compartments (Figure 2C, arrowheads). Given that these observations were made on ultrathin cryosections (0.2 µm thick), this suggested that such association was not random and was probably multiple; each virus-containing compartment having multiple points of contacts with adjacent microtubule bundle(s).

Thus in HIV-infected macrophages, microtubules appeared to play a key role in the intracellular transport of Gag+ compartments containing viral particles.

### Example 3 The kinesin KIF3A associates with microtubules at the limiting membrane of Gag+ compartments

If transport of Gag+ compartments occurs along microtubules then molecular motors such as kinesins or dyneins must be involved. The kinesin KIF3C has been identified in a genomic functional screen performed with HeLa-derived TZM-bl reporter cells, as essential for the HIV-1 late cycle ²⁰. In another genome-wide screen using HEK293T cells, KIF3A was found to be involved in the viral cycle ²². KIF3A can form heterodimers either with KIF3B or KIF3C to walk along microtubules towards their plus end ^{23,24}. Therefore we investigated whether KIF3A was involved in the HIV cycle in macrophages. We first analyzed KIF3A intracellular distribution in HIV-infected macrophages. We observed an association between KIF3A and Gag+ compartments (Figure 3A). While Gag+ compartments were heterogenous in size and often rather large (several µm of diameter), KIF3A staining tended to be limited in one or several zones at the periphery of the Gag+ compartments. 3D reconstruction was needed to better appreciate this association (Figure 3C). Thus, patches of KIF3A staining were found to associate both with microtubules and with Gag+ compartments. Env co-staining revealed that many of the Gag+KIF3A+ compartments were also Env+ (not shown), suggesting that they contained viral particles.

Next, we analyzed the effects of the disruption of the microtubule network on KIF3A distribution. Nocodazole exposure of HIV-1-infected macrophages led to the expected dissociation of the microtubule network and the redistribution of Gag and Gag+ compartments towards one pole of the nucleus (Figure 3B). Interestingly, the majority of KIF3A staining also became concentrated together with Gag+ structures at one pole of the nucleus, while some surface KIF3A staining clearly remained at the cell surface (Figure 3B). Wash out of the nocodazole during 15 min allowed growth of microtubules, which was accompanied by spreading of the Gag+ compartments and of KIF3A (data not shown).

Thus, our data indicated that KIF3A was involved in the transport of Gag+ compartments containing virions along microtubules.

### Example 4 HIV production by macrophages, but not by T cells, is KIF3A-dependent

The role of KIF3A in HIV-infected macrophages was evaluated by RNAi on the p24 production (see experimental design Figure 4A). Using 3 different siRNA specific for KIF3A that had been validated in HeLa cells, we induced in primary macrophages the depletion of KIF3A, which efficiency ranged from 50% to 80% as judged by western blot analysis (Figure 4B). Importantly, KIF3A depletion by RNAi led to a strong reduction (up to 75%) of the amounts of p24 secreted in the supernatant (Figure 4C). A reduction of the amoint of secreted p24 was also obtained when using siRNA against KIF5B (data not shown). The extent of the inhibition was similar to the one obtained when knocking down the expression of the ESCRT protein Tsg101, which is crucial for viral budding in 293T cells ⁷. In contrast, KIF4, a kinesin reported to interact with Gag and to be required during the HIV-1 cycle in COS cells ²⁵ appeared to be dispensable as its knock down did not reduce HIV-1 production in macrophages (Figure 4C). We checked that cell viability (Figure 4D), and percentages of infected macrophages (Figure 4E) were similar in the different cell populations upon transfection and infection. Importantly, early steps of infection were not affected by KIF3A depletion. This was measured using the TZM-bl reporter cell line, which was transfected with the various siRNA, HIV-infected and assayed 24 hr p.i. for β-gal activity which expression is driven by a Tat sensitive promoter (Figure 4F). In addition, no major difference in the infectivity of the virions still produced by macrophages under these conditions was observed when using normalized amounts of p24 (Figure 4G).

To evaluate whether the involvement of KIF3A in the HIV-1 cycle was cell type specific, we performed similar RNAi experiments in HIV-1 (NL4-3)-infected T cells. The experimental design was slightly different as the kinetics of replication of HIV is faster in this cell type. Moreover, we had to use lentiviruses for delivery of shRNA for efficient knock-down of KIF3A expression in Jurkat T cells (Figure S3). We observed that the production of p24 by infected Jurkat T cells was totally independent of KIF3A expression (Figure S3). Like in macrophages, cell viability and infectivity of the virus produced were not affected by KIF3A depletion (Figure S3).

Thus, KIF3A was required for the late phases of the HIV-1 cycle in macrophages but not in T cells.

### Example 5 Intracellular trafficking of the virus-containing compartments is driven by KIF3A

To further elucidate the role of KIF3A, we investigated the dynamics of KIF3A in infected macrophages. Kinesins have often been tagged by replacing the cargo domain with a fluorescent protein ¹⁹. However, if Gag is indeed a cargo ofKIF3A, use of such a fusion protein may prevent definitive conclusions regarding the role of KIF3A with respect to Gag. Here, we used an original construct where the fluorescent protein mCherry has been inserted between the stalk and the cargo domains of KIF3A, flanked by two spacers to provide flexibility (Figure 5A). This KIF3A-internal mCherry construct (KIF3A-iCh) was well expressed as seen by western blot and immunofluorescence in 293T cells (Figure S4) using an antibody specific for the 20 last amino acids of KIF3A (i.e. within the cargo domain). This was in contrast to other KIF3A construcs that we derived and which were not correctly expressed (Figure S4). The KIF3A-iCh fusion protein was observed to move on microtubules (identified by co-expression of EGFP-α-tubulin) at a speed of about 0.5 µm/sec, as expected for kinesin-like movements ^{26,27}. Moreover, we observed that KIF3A-iCh colocalized with CD81 in 293 T cells (Figure S4) and with one of its putative cargo protein, the protease MT1-MMP ²⁸ (not shown) suggesting that KIF3A-iCh properly behaved like the wild type protein.

Then, to further investigate the relationship between Gag and KIF3A during infection, primary macrophages were co-infected with HIV Gag-iGFP virus and KIF3A-iCh lentiviral vector. After 7 days of infection, macrophages were fixed on coverslips with etched grids with coordinates and imaged by confocal microscopy. Co-expression of both fluorescent proteins was visualized in large intracellular compartments (Figure 5B). Epon embedding followed by sectioning of the samples allowed to perform EM of the very same cell previously imaged by fluorescence microscopy. At low magnification, EM revealed the position of the virus-containing compartments within the cell (Figure 5C). At large magnification, the compartments corresponding to the Gag-iGFP+ KIF3A-iCh+ ones, contained numerous viral particles (Figure 5D). These compartments were active in term of HIV assembly, as budding profiles were visible at their limiting membrane (Figure 5D, arrowheads). They contained both immature and mature viral particles.

In a parallel set of experiments, primary macrophages co-infected with HIV Gag-iGFPΔEnv virus and KIF3A-iCh lentiviral vector were prepared for time-lapse microscopy. After 5 days of infection, acquisition of several planes (step size = 0.5 µm) was performed every 5 min for 5 hr by spinning disk microscopy (not shown). Observation of the 4D movie at time 0 (Figure 5E) revealed that KIF3A-iCh was associated in a polarized manner with the periphery of Gag-iGFP+ compartments, in agreement with our previous observations regarding endogenous KIF3A distribution in HIV-1-infected macrophages (Figure 3). Moreover, time-lapse revealed that both structures stayed associated with time, moved together, and possibly travelled on microtubule tracks (Figure 5E, see tracking).

Thus, our data indicated that KIF3A drove the transport of Gag+ compartments containing virions in macrophages.

### References

1. Sharova N, Swingler C, Sharkey M, Stevenson M. Macrophages archive HIV-1 virions for dissemination in trans. Embo J. 2005;24:2481-2489.
2. Chun TW, Engel D, Mizell SB, et al. Effect of interleukin-2 on the pool of latently infected, resting CD4+ T cells in HIV-1-infected patients receiving highly active anti-retroviral therapy. Nat Med. 1999;5:651-655.
3. Zhu T, Muthui D, Holte S, et al. Evidence for human immunodeficiency virus type 1 replication in vivo in CD14(+) monocytes and its potential role as a source of virus in patients on highly active antiretroviral therapy. J Virol. 2002;76:707-716.
4. van Lunzen J, Hoffmann C. Virological rebound and its consequences during treatment interruption. Curr Opin HIV AIDS. 2007;2:1-5.
5. Alexaki A, Liu Y, Wigdahl B. Cellular reservoirs of HIV-1 and their role in viral persistence. Curr HIV Res. 2008;6:388-400.
6. Hurley JH, Hanson PI. Membrane budding and scission by the ESCRT machinery: it's all in the neck. Nat Rev Mol Cell Biol; 1:556-566.
7. Garrus JE, von Schwedler UK, Pornillos OW, et al. Tsg101 and the vacuolar protein sorting pathway are essential for hiv-1 budding. Cell. 2001;107:55-65.
8. Welsch S, Habermann A, Jager S, Muller B, Krijnse-Locker J, Krausslich HG. Ultrastructural analysis of ESCRT proteins suggests a role for endosome-associated tubular-vesicular membranes in ESCRT function. Traffic. 2006;7:1551-1566.
9. Benaroch P, Billard E, Gaudin R, Schindler M, Jouve M. HIV-1 assembly in macrophages. Retrovirology. 2010;7:29.
10. Raposo G, Moore M, Innes D, et al. Human Macrophages Accumulate HIV-1 Particles in MHC II Compartments. Traffic. 2002;3:718-729.
11. Pelchen-Matthews A, Kramer B, Marsh M. Infectious HIV-1 assembles in late endosomes in primary macrophages. J Cell Biol. 2003;162:443-455.
12. Jouve M, Sol-Foulon N, Watson S, Schwartz O, Benaroch P. HIV-1 Buds and Accumulates in "Nonacidic" Endosomes of Macrophages. Cell Host Microbe. 2007;2:85-95.
13. Kramer B, Pelchen-Matthews A, Deneka M, Garcia E, Piguet V, Marsh M. HIV interaction with endosomes in macrophages and dendritic cells. Blood Cells Mol Dis. 2005;35:136-142.
14. Deneka M, Pelchen-Matthews A, Byland R, Ruiz-Mateos E, Marsh M. In macrophages, HIV-1 assembles into an intracellular plasma membrane domain containing the tetraspanins CD81, CD9, and CD53. J Cell Biol. 2007;177:329-341.
15. Welsch S, Keppler OT, Habermann A, Allespach I, Krijnse-Locker J, Krausslich HG. HIV-1 Buds Predominantly at the Plasma Membrane of Primary Human Macrophages. PLoS Pathog. 2007;3:e36.
16. Bennett AE, Narayan K, Shi D, et al. Ion-abrasion scanning electron microscopy reveals surface-connected tubular conduits in HIV-infected macrophages. PLOS Pathogens. 2009;in press.
17. Jolly C, Mitar I, Sattentau QJ. Requirement for an intact T-cell actin and tubulin cytoskeleton for efficient assembly and spread of human immunodeficiency virus type 1. J Virol. 2007;81:5547-5560.
18. Nishi M, Ryo A, Tsurutani N, et al. Requirement for microtubule integrity in the SOCS1-mediated intracellular dynamics of HIV-1 Gag. FEBS Lett. 2009;583:1243-1250.
19. Hirokawa N, Noda Y, Tanaka Y, Niwa S. Kinesin superfamily motor proteins and intracellular transport. Nat Rev Mol Cell Biol. 2009;10:682-696.
20. Brass AL, Dykxhoorn DM, Benita Y, et al. Identification of host proteins required for HIV infection through a functional genomic screen. Science. 2008;319:921-926.
21. Orenstein JM. Ultrastructure of HIV/AIDS. Ultrastruct Pathol. 2002;26:245-250.
22. König R, Zhou Y, Elleder D, et al. Global analysis of host-pathogen interactions that regulate early-stage HIV-1 replication. Cell. 2008;135:49-60.
23. Yamazaki H, Nakata T, Okada Y, Hirokawa N. KIF3A/B: a heterodimeric kinesin superfamily protein that works as a microtubule plus end-directed motor for membrane organelle transport. J Cell Biol. 1995;130:1387-1399.
24. Yang Z, Goldstein LS. Characterization of the KIF3C neural kinesin-like motor from mouse. Mol Biol Cell. 1998;9:249-261.
25. Martinez NW, Xue X, Berro RG, Kreitzer G, Resh MD. Kinesin KIF4 regulates intracellular trafficking and stability of the human immunodeficiency virus type 1 Gag polyprotein. J Virol. 2008;82:9937-9950.
26. Cai D, McEwen DP, Martens JR, Meyhofer E, Verhey KJ. Single molecule imaging reveals differences in microtubule track selection between Kinesin motors. PLoS Biol. 2009;7:e1000216.
27. Loubery S, Wilhelm C, Hurbain I, Neveu S, Louvard D, Coudrier E. Different microtubule motors move early and late endocytic compartments. Traffic. 2008;9:492-509.
28. Wiesner C, Faix J, Himmel M, Bentzien F, Linder S. KIF5B and KIF3A/KIF3B kinesins drive MT1-MMP surface exposure, CD44 shedding, and extracellular matrix degradation in primary macrophages. Blood; 116:1559-1569.
29. Hubner W, Chen P, Del Portillo A, Liu Y, Gordon RE, Chen BK. Sequence of human immunodeficiency virus type 1 (HIV-1) Gag localization and oligomerization monitored with live confocal imaging of a replication-competent, fluorescently tagged HIV-1. J Virol. 2007;81:12596-12607.
30. Martin-Serrano J, Zang T, Bieniasz PD. HIV-1 and Ebola virus encode small peptide motifs that recruit Tsg101 to sites of particle assembly to facilitate egress. Nat Med. 2001;7:1313-1319.

## Claims

1. A kinesin inhibitor for use in the treatment of HIV infection.

2. The kinesin inhibitor of claim 1, wherein said kinesin inhibitor is a N-type kinesin inhibitor, preferably a kinesin-1 inhibitor and/or a kinesin-2 inhibitor.

3. The kinesin inhibitor of any one of claims 1 or 2, wherein said inhibitor is selective for said kinesin, and preferably acts through direct binding to said kinesin.

4. The kinesin inhibitor of any one of claim 1 to 3, wherein said kinesin inhibitor is a KIF3A inhibitor and/or a KIF5B inhibitor.

5. The kinesin inhibitor according to any one of claim 1 to 4, wherein said inhibitor is selected from the group consisting of a small molecule, a kinesin aptamer, an anti-kinesin antibody, a nucleic acid or a dominant negative peptide.

6. The kinesin inhibitor according to any one of claims 1 to 5, wherein said inhibitor inhibits the expression of said kinesin, and is preferably selected from the group consisting of an antisense nucleic acid, a ribozyme and an interfering RNA.

7. A pharmaceutical composition comprising at least one kinesin inhibitor according to any one of the preceding claims for use in the treatment of HIV infection.

8. The composition of claim 7, further comprising at least another anti-viral treatment, preferably an anti-VIH drug used in highly active antiretroviral therapy (HAART).

9. A tagged kinesin comprising a kinesin motor domain, a stalk domain, a cargo domain and a tag allowing said tagged kinesin to be detected, and wherein said tag is located between said stalk domain and said cargo domain.

10. The tagged kinesin of claim 9, wherein said kinesin motor domain, stalk domain and cargo domain all belong to the same kinesin, preferably a N-type kinesin, more preferably a kinesin 1 or to a kinesin 2, even more preferably to Kif3A or to KIF5B

11. The tagged kinesin according to any one of claim 9 or 10, wherein said tag is a fluorescent tag protein, more preferably a mCherry protein

12. The tagged kinesin according to any one of claims 9 to 11, wherein said tag is flanked at least on one side, preferably on both sides, by spacers providing flexibility.

13. The tagged kinesin according to any one of claims 9 to 12, comprising the amino acid sequence of SEQ ID N°4.

14. A nucleic acid sequence comprising a sequence which codes for a tagged kinesin according to any one of the preceding claim 9 to 13.

15. Use of a tagged kinesin according to any one of claim 9 to 13 to screen for kinesin inhibitors.

16. A method of screening for compounds useful for the treatment of HIV infection comprising the steps of:
a) providing or obtaining a candidate compound; and
b) determining whether said candidate compound inhibits the activity of expression of a kinesin, preferably a N-type kinesin, more preferably a kinesin-1 or a kinesin-2 , even more preferably kinesin KIF3A and/or KIF5B,
wherein the ability of said candidate compound to inhibits the expression or activity of said kinesin indicates that said candidate compound is indicative of its usefulness for the treatment of HIV infection.

17. The screening method of claim 16 further comprising, for such candidate compound having the ability to inhibit the expression and/or activity of a kinesin of interest, a step c) comprising determining whether said candidate compound is able to inhibit and/or decrease HIV infection.,

18. The screening method of claim 17, wherein said step c) comprises determining whether said candidate compound interferes with HIV virions formation, preferably using primary macrophages/monocytes or macrophage/monocyte cell lines infected with HIV.

19. The screening method of claim 16, wherein step b) comprises determining whether said candidate compound inhibits the activity of a tagged kinesin according to any one of claims 9 to 13.
